(19) Europäisches Patentamt
European Patent Office
Office européen des brevets

(11) Veröffentlichungsnummer: **0 278 311 B1**

# (12) EUROPÄISCHE PATENTSCHRIFT

(45) Veröffentlichungstag der Patentschrift: **18.07.90**

(51) Int. Cl.[5]: **C07D 307/14**, C07D 405/06, A01N 43/08, A01N 43/40

(21) Anmeldenummer: **88101077.1**

(22) Anmeldetag: **26.01.88**

(54) **2-Aminomethyltetrahydrofurane.**

(30) Priorität: **07.02.87 DE 3703876**
**17.09.87 DE 3731198**

(43) Veröffentlichungstag der Anmeldung:
**17.08.88 Patentblatt 88/33**

(45) Bekanntmachung des Hinweises auf die Patenterteilung:
**18.07.90 Patentblatt 90/29**

(84) Benannte Vertragsstaaten:
**BE CH DE FR GB IT LI NL**

(56) Entgegenhaltungen:
**EP-A- 0 068 331**
**DE-A- 3 413 996**
**US-A- 3 532 722**

**Chemical Abstracts, Band 79, Nr.9, 3. September 1973, Columbus, Ohio, USA. Monkovic I.; Perron Y.G.; Martel R.; Simpson W.J.; Gylys J.A.; "Substituted tetrahydrofurfurylamines as potential anti-depressants." Seite 8, Spalte 1, Zusammenfassung-Nr. 49 090z & J. Med. Chem. 1973, 16(4), 403-7 000**

**Die Akte enthält technische Angaben, die nach dem Eingang der Anmeldung eingereicht wurden und die nicht in dieser Patentschrift enthalten sind.**

(73) Patentinhaber: **BAYER AG,**
**D-5090 Leverkusen 1 Bayerwerk(DE)**

(72) Erfinder: **Weissmüller, Joachim, Dr.,**
**Carl-Langhans-Strasse 53, D-4019 Monheim(DE)**
Erfinder: **Berg, Dieter, Dr., Gellertweg 27,**
**D-5600 Wuppertal 1(DE)**
Erfinder: **Dutzmann, Stefan, Dr., Leinenweberweg 33,**
**D-4000 Düsseldorf 13(DE)**
Erfinder: **Hänssler, Gerd, Dr., Am Arenzberg 58a,**
**D-5090 Leverkusen 3(DE)**

**EP 0 278 311 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue 2-Aminomethyltetrahydrofurane, mehrere Verfahren zu ihrer Herstellung und ihre Verwendung als Schädlingsbekämpfungsmittel.

Es ist bereits bekannt, daß bestimmte Aminomethyltetrahydrofurane, wie beispielsweise das 2-(4-Chlorphenyl)5-(3,5-dimethylpiperidin-1-yl-methyl)-tetrahydrofuran fungizide Eigenschaften besitzen (vgl. DE-OS 3 413 996).

Die Wirksamkeit dieser vorbekannten Verbindungen ist jedoch insbesondere bei niedrigen Aufwandmengen und Konzentrationen nicht in allen Anwendungsgebieten völlig zufriedenstellend.

Weiterhin sind bekannt Aryl-substituierte Aminomethyltetrahydrofurane, die in der Medizin Verwendung finden (vgl. EP-A 68 331).

Es wurden neue 2-Aminomethyltetrahydrofurane der allgemein Formel (I),

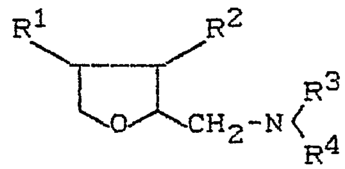

(I)

in welcher

$R^1$ für gegebenenfalls einfach bis mehrfach, gleich oder verschieden substituiertes Phenyl steht, wobei als Substituenten infrage kommen: Halogen, Cyano, Nitro, jeweils geradkettiges oder verzweigtes Alkyl, Alkoxy, Alkylthio, Halogenalkyl, Halogenalkoxy oder Halogenalkylthio mit jeweils 1 bis 6 Kohlenstoffatomen und gegebenenfalls 1 bis 9 gleichen oder verschiedenen Halogenatomen, Amino, jeweils geradkettiges oder verzweigtes Alkylamino, Dialkylamino oder Alkoximinoalkyl mit jeweils 1 bis 4 Kohlenstoffatomen in den einzelnen Alkylteilen; ferner für jeweils gegebenenfalls einfach bis mehrfach, gleich oder verschieden substituiertes α-Naphthyl oder β-Naphthyl steht, wobei als Substituenten jeweils infrage kommen: Halogen, jeweils geradkettiges oder verzweigtes Alkyl oder Alkoxy mit jeweils 1 bis 4 Kohlenstoffatomen; weiterhin für gegebenenfalls einfach bis mehrfach, gleich oder verschieden substituiertes Cycloalkyl mit 3 bis 7 Kohlenstoffatomen steht, wobei als Substituenten infrage kommen jeweils geradkettiges oder verzweigtes Alkyl, Alkoxy, Halogenalkyl oder Halogenalkoxy mit jeweils 1 bis 6 Kohlenstoffatomen und gegebenenfalls 1 bis 9 gleichen oder verschiedenen Halogenatomen;

ferner für jeweils gegebenenfalls einfach bis mehrfach, gleich oder verschieden substituiertes Tetrahydronaphthyl oder Decahydronaphthyl steht, wobei als Substituenten für die hydrierten Ringe infrage kommen: geradkettiges oder verzweigtes Alkyl und Alkoxy mit jeweils 1 bis 6 Kohlenstoffatomen, sowie zusätzlich für die aromatischen Ringe Halogen;

und schließlich für einen gegebenenfalls einfach bis mehrfach, gleich oder verschieden durch Alkyl mit 1 bis 6 Kohlenstoffatomen und/oder Halogen substituierten 5- oder 6-gliedrigen Heteroarylrest mit 1 oder 2 Heteroatomen, insbesondere Stickstoff, Sauerstoff oder Schwefel steht;

$R^2$ für Wasserstoff oder Methyl steht und

$R^3$ und $R^4$ unabhängig voneinander jeweils für Wasserstoff; für jeweils geradkettiges oder verzweigtes Alkyl mit 1 bis 12 Kohlenstoffatomen, Alkenyl mit 3 bis 8 Kohlenstoffatomen, Alkinyl mit 3 bis 8 Kohlenstoffatomen, Hydroxyalkyl mit 2 bis 6 Kohlenstoffatomen, Alkoxyalkyl oder Dialkoxyalkyl mit jeweils 1 bis 8 Kohlenstoffatomen oder Hydroxyalkoxyalkyl mit 2 bis 8 Kohlenstoffatomen in den einzelnen Alkylteilen; für jeweils geradkettiges oder verzweigtes Dioxolanylalkyl, Oxolanylalkyl oder Dioxanylalkyl mit jeweils 1 bis 4 Kohlenstoffatomen im Alkylteil oder für jeweils gegebenenfalls im Cycloalkylteil einfach bis mehrfach, gleich oder verschieden substituiertes Cycloalkyl oder Cycloalkylalkyl mit jeweils 3 bis 7 Kohlenstoffatomen im Cycloalkylteil und gegebenenfalls 1 bis 4 Kohlenstoffatomen im geradkettigen oder verzweigten Alkylteil stehen, wobei als Substituenten jeweils infrage kommen:

Halogen, jeweils geradkettiges oder verzweigtes Alkyl, Alkoxy, Halogenalkyl oder Halogenalkoxy mit jeweils 1 bis 4 Kohlenstoffatomen und gegebenenfalls 1 bis 9 gleichen oder verschiedenen Halogenatomen; außerdem für jeweils gegebenenfalls einfach bis mehrfach, gleich oder verschieden im Arylteil substituiertes Arylalkyl, Arylalkenyl oder Aryl mit jeweils 6 bis Kohlenstoffatomen im Arylteil und gegebenenfalls mit bis zu 6 Kohlenstoffatomen im geradkettigen oder verzweigten Alkyl- bzw. Alkenylteil stehen, wobei als Arylsubstituenten jeweils infrage kommen:

Halogen, Cyano, Nitro, jeweils geradkettiges oder verzweigtes Alkyl, Alkoxy, Alkylthio, Halogenalkyl, Halogenalkoxy, Halogenalkylthio oder Alkoximinoalkyl mit jeweils 1 bis 4 Kohlenstoffatomen in den einzelnen Alkylteilen und gegebenenfalls 1 bis 9 gleichen oder verschiedenen Halogenatomen; oder

$R^3$ und $R^4$ gemeinsam mit dem Stickstoffatom, an welches sie gebunden sind, für einen gegebenenfalls substituierten, gesättigten Heterocyclus stehen, der gegebenenfalls weitere Heteroatome, insbesondere Stickstoff, Sauerstoff oder Schwefel enthalten kann, wobei als Substituenten infrage kommen: jeweils geradkettiges oder verzweigtes Alkyl oder Hydroxyalkyl mit jeweils 1 bis 4 Kohlenstoffatomen,

und deren Säureadditionssalze.

Die Verbindungen der Formel (I) können als geometrische und/oder optische Isomere oder Isomerengemische unterschiedlicher Zusammensetzung vorliegen. Sowohl die reinen Isomeren als auch die Isomerengemische werden erfindungsgemäß beansprucht.

Weiterhin wurde gefunden, daß man die neuen 2-Aminomethyltetrahydrofurane der allgemeinen Formel (I),

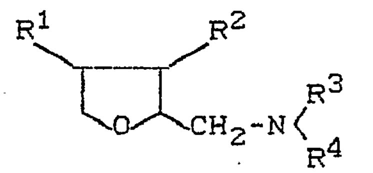

(I)

in welcher

$R^1$ für gegebenenfalls einfach bis mehrfach gleich oder verschieden substituiertes Phenyl steht, wobei als Substituenten infrage kommen: Halogen, Cyano, Nitro, jeweils geradkettiges oder verzweigtes Alkyl, Alkoxy, Alkylthio, Halogenalkyl, Halogenalkoxy oder Halogenalkylthio mit jeweils 1 bis 6 Kohlenstoffatomen und gegebenenfalls 1 bis 9 gleichen oder verschiedenen Halogenatomen, Amino, jeweils geradkettiges oder verzweigtes Alkylamino, Dialkylamino oder Alkoximinoalkyl mit jeweils 1 bis 4 Kohlenstoffatomen in den einzelnen Alkylteilen; ferner für jeweils gegebenenfalls einfach bis mehrfach, gleich oder verschieden substituiertes α-Naphthyl oder β-Naphthyl steht, wobei als Substituenten jeweils infrage kommen: Halogen, jeweils geradkettiges oder verzweigtes Alkyl oder Alkoxy mit jeweils 1 bis 4 Kohlenstoffatomen; weiterhin für gegebenenfalls einfach bis mehrfach, gleich oder verschieden substituiertes Cycloalkyl mit 3 bis 7 Kohlenstoffatomen steht, wobei als Substituenten infrage kommen: jeweils geradkettiges oder verzweigtes Alkyl, Alkoxy, Halogenalkyl oder Halogenalkoxy mit jeweils 1 bis 6 Kohlenstoffatomen und gegebenenfalls 1 bis 9 gleichen oder verschiedenen Halogenatomen;

ferner für jeweils gegebenenfalls einfach bis mehrfach, gleich oder verschieden substituiertes Tetrahydronaphthyl oder Decahydronaphthyl steht, wobei als Substituenten für die hydrierten Ringe infrage kommen: geradkettiges oder verzweigtes Alkyl und Alkoxy mit jeweils 1 bis 6 Kohlenstoffatomen, sowie zusätzlich für die aromatischen Ringe Halogen;

und schließlich für einen gegebenenfalls einfach bis mehrfach, gleich oder verschieden durch Alkyl mit 1 bis 6 Kohlenstoffatomen und/oder Halogen substituierten 5- oder 6-gliedrigen Heteroarylrest mit 1 oder 2 Heteroatomen, insbesondere Stickstoff, Sauerstoff oder Schwefel steht;

$R^2$ für Wasserstoff oder Methyl steht und

$R^3$ und $R^4$ unabhängig voneinander jeweils für Wasserstoff; für jeweils geradkettiges oder verzweigtes Alkyl mit 1 bis 12 Kohlenstoffatomen, Alkenyl mit 3 bis 8 Kohlenstoffatomen, Alkinyl mit 3 bis 8 Kohlenstoffatomen, Hydroxyalkyl mit 2 bis 6 Kohlenstoffatomen, Alkoxyalkyl oder Dialkoxyalkyl mit jeweils 1 bis 8 Kohlenstoffatomen oder Hydroxyalkoxyalkyl mit 2 bis 8 Kohlenstoffatomen in den einzelnen Alkylteilen; für jeweils geradkettiges oder verzweigtes Dioxolanylalkyl, Oxolanylalkyl oder Dioxanylalkyl mit jeweils 1 bis 4 Kohlenstoffatomen im Alkylteil oder für jeweils gegebenenfalls im Cycloalkylteil einfach bis mehrfach, gleich oder verschieden substituiertes Cyclcalkyl oder Cycloalkylalkyl mit jeweils 3 bis 7 Kohlenstoffatomen im Cycloalkylteil und gegebenenfalls 1 bis 4 Kohlenstoffatomen im geradkettigen oder verzweigten Alkylteil stehen, wobei als Substituenten jeweils infrage kommen:

Halogen, jeweils geradkettiges oder verzweigtes Alkyl, Alkoxy, Halogenalkyl oder Halogenalkoxy mit jeweils 1 bis 4 Kohlenstoffatomen und gegebenenfalls 1 bis 9 gleichen oder verschiedenen Halogenatomen; außerdem für jeweils gegebenenfalls einfach bis mehrfach, gleich oder verschieden im Arylteil substituiertes Arylalkyl, Arylalkenyl oder Aryl mit jeweils 6 bis 10 Kohlenstoffatomen im Arylteil und gegebenenfalls mit bis zu 6 Kohlenstoffatomen im geradkettigen oder verzweigten Alkyl- bzw. Alkenylteil stehen, wobei als Arylsubstituenten jeweils infrage kommen:

Halogen, Cyano, Nitro, jeweils geradkettiges oder verzweigtes Alkyl, Alkoxy, Alkylthio, Halogenalkyl, Halogenalkoxy, Halogenalkylthio oder Alkoximinoalkyl mit jeweils 1 bis 4 Kohlenstoffatomen in den einzelnen Alkylteilen und gegebenenfalls 1 bis 9 gleichen oder verschiedenen Halogenatomen; oder

$R^3$ und $R^4$ gemeinsam mit dem Stickstoffatom, an welches sie gebunden sind, für einen gegebenenfalls substituierten, gesättigten Heterocyclus stehen, der gegebenenfalls weitere Heteroatome, insbesondere Stickstoff, Sauerstoff oder Schwefel enthalten kann, wobei als Substituenten infrage kommen: jeweils geradkettiges oder verzweigtes Alkyl oder Hydroxyalkyl mit jeweils 1 bis 4 Kohlenstoffatomen, sowie deren pflanzenverträgliche Säureadditionssalze, deren geometrische und optische Isomere und Isomerengemische erhält, wenn man

(a) substituierte Tetrahydrofurane der Formel (II),

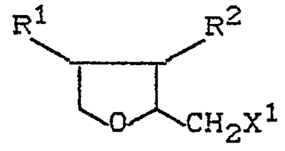

(II)

in welcher

$R^1$ und $R^2$ die oben angegebene Bedeutung haben und

$X^1$ für eine elektronenanziehende Abgangsgruppe steht,

mit Aminen der Formel (III),

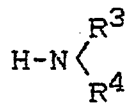

(III)

in welcher

$R^3$ und $R^4$ die oben angegebene Bedeutung haben, gegebenenfalls in Gegenwart eines Verdünnungsmittels und gegebenenfalls in Gegenwart eines Säurebindemittels umsetzt; oder wenn man

(b) die nach Verfahren (a) und/oder (c) erhältlichen Aminomethyltetrahydrofurane der Formel (Ia),

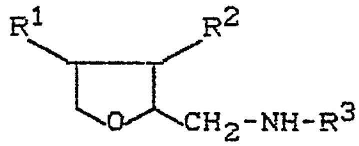

(Ia)

in welcher

$R^1$, $R^2$ und $R^3$ die oben angegebene Bedeutung haben,

mit Alkylierungsmitteln der Formel (IV),

$$R^{4-1}-X^2 \qquad (IV)$$

in welcher

$R^{4-1}$ für jeweils geradkettiges oder verzweigtes Alkyl mit 1 bis 12 Kohlenstoffatomen, Alkenyl mit 3 bis 8 Kohlenstoffatomen, Alkinyl mit 3 bis 8 Kohlenstoffatomen, Hydroxyalkyl mit 2 bis 6 Kohlenstoffatomen, Alkoxyalkyl, Diakoxyalkyl oder Hydroxyalkoxyalkyl mit jeweils 1 bis 6 Kohlenstoffatomen in den einzelnen Alkylteilen, für Dioxolanylalkyl, Oxolanylalkyl oder Dioxanylalkyl mit jeweils 1 bis 4 Kohlenstoffatomen im geradkettigen oder verzweigten Alkylteil, für jeweils gegebenenfalls im Cycloalkylteil einfach bis mehrfach, gleich oder verschieden substituiertes Cycloalkyl oder Cycloalkylalkyl mit jeweils 3 bis 7 Kohlenstoffatomen im Cycloalkylteil und gegebenenfalls 1 bis 4 Kohlenstoffatomen im geradkettigen oder verzweigten Alkylteil, wobei als Substituenten jeweils infrage kommen: Halogen, jeweils geradkettiges oder verzweigtes Alkyl, Alkoxy, Halogenalkyl oder Halogenalkoxy mit jeweils 1 bis 4 Kohlenstoffatomen und gegebenenfalls 1 bis 9 gleichen oder verschiedenen Halogenatomen; außerdem für jeweils gegebenenfalls einfach bis mehrfach, gleich oder verschieden im Arylteil substituiertes Arylalkyl oder Arylalkenyl mit jeweils 6 bis 10 Kohlenstoffatomen im Arylteil und bis zu 6 Kohlenstoffatomen im geradkettigen oder verzweigten Alkyl- bzw., Alkenylteil, wobei als Arylsubstituenten jeweils infrage kommen: Halogen, Cyano, Nitro, jeweils geradkettiges oder verzweigtes Alkyl, Alkoxy, Alkylthio, Halogenalkyl, Halogenalkoxy, Halogenalkylthio, Alkoxycarbonyl oder Alkoximinoalkyl mit jeweils 1 bis 4 Kohlenstoffatomen in den einzelnen Alkylteilen und gegebenenfalls 1 bis 9 gleichen oder verschiedenen Halogenatomen;

und

$X^2$ für eine elektronenanziehende Abgangsgruppe steht, gegebenenfalls in Gegenwart eines Verdünnungsmittels und gegebenenfalls in Gegenwart eines Säurebindemittels umsetzt; oder wenn man

(c) die nach Verfahren (a) erhältlichen Aminomethyltetrahydrofurane der Formel (Ib)

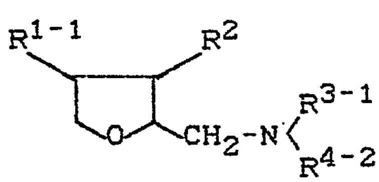

(Ib)

in welcher

$R^{1-1}$ für gegebenenfalls einfach bis mehrfach, gleich oder verschieden substituiertes Phenyl, wobei als Substituenten infrage kommen: Halogen, Cyano, Nitro, jeweils; geradkettiges oder verzweigtes Alkyl, Alkoxy, Alkylthio, Halogenalkyl, Halogenalkoxy oder Halogenalkylthio mit jeweils 1 bis 6 Kohlenstoffatomen und gegebenenfalls 1 bis 9 gleichen oder verschiedenen Halogenatomen, Amino, jeweils geradkettiges oder verzweigtes Alkylamino, Dialkylamino oder Alkoximinoalkyl mit jeweils 1 bis 4 Kohlenstoffatomen in den einzelnen Alkylteilen; ferner für jeweils gegebenenfalls einfach bis mehrfach, gleich oder verschieden substituiertes α-Naphthyl oder β-Naphthyl, wobei als Substituenten jeweils infrage kommen: Halogen, jeweils geradkettiges oder verzweigtes Alkyl oder Alkoxy mit jeweils 1 bis 4 Kohlenstoffatomen;

$R^2$ für Wasserstoff oder Methyl;

$R^{3-1}$ und $R^{4-2}$ unabhängig voneinander für Wasserstoff; für jeweils geradkettiges oder verzweigtes Alkyl mit 1 bis 12 Kohlenstoffatomen, Hydroxyalkyl mit 2 bis 6 Kohlenstoffatomen, Alkoxyalkyl oder Dialkoxyalkyl mit jeweils 1 bis 8 Kohlenstoffatomen oder Hydroxyalkoxyalkyl mit 2 bis 8 Kohlenstoffatomen in den einzelnen Alkylteilen; für jeweils geradkettiges oder verzweigtes Dioxolanylalkyl, Oxolanylalkyl oder Dioxanylalkyl mit jeweils 1 bis 4 Kohlenstoffatomen im Alkylteil oder für jeweils gegebenenfalls im Cycloalkylteil einfach oder mehrfach, gleich oder verschieden substituiertes Cycloalkyl oder Cycloalkylalkyl mit jeweils 3 bis 7 Kohlenstoffatomen im Cycloalkylteil und gegebenenfalls 1 bis 4 Kohlenstoffatomen im geradkettigen oder verzweigten Alkylteil stehen, wobei als Substituenten jeweils infrage kommen:

Halogen, jeweils geradkettiges oder verzweigtes Alkyl, Alkoxy, Halogenalkyl oder Halogenalkoxy mit jeweils 1 bis 4 Kohlenstoffatomen und gegebenenfalls 1 bis 9 gleichen oder verschiedenen Halogenatomen; außerdem für jeweils gegebenenfalls einfach bis mehrfach, gleich oder verschieden im Arylteil substituiertes Arylalkyl oder Aryl mit jeweils 6 bis 10 Kohlenstoffatomen im Arylteil und gegebenenfalls mit bis zu 6 Kohlenstoffatomen im geradkettigen oder verzweigten Alkylteil, wobei als Arylsubstituenten jeweils die oben genannten Cycloalkylsubstituenten infrage kommen; oder

$R^{3-1}$ und $R^{4-2}$ gemeinsam mit dem Stickstoffatom, an welches sie gebunden sind, für einen gegebenenfalls einfach bis mehrfach, gleich oder verschieden substituierten, gesättigten 5- bis 7 gliedrigen Heterocyclus, der gegebenenfalls ein weiteres Heteroatom, insbesondere Stickstoff, Sauerstoff oder Schwefel enthalten kann, wobei als Substituenten infrage kommen: jeweils geradkettiges oder verzweigtes Alkyl oder Hydroxyalkyl mit jeweils 1 bis 4 Kohlenstoffatomen gegebenenfalls in Gegenwart eines Verdünnungsmittels und gegebenenfalls in Gegenwart eines Katalysators sowie gegebenenfalls unter erhöhtem Druck vorhandene Arylsubstituenten hydriert;

und gegebenenfalls anschließend eine Säure addiert.

Schließlich wurde gefunden, daß die neuen Aminomethyltetrahydrofurane der allgemeinen Formel (I) eine Wirkung gegen Schädlinge, insbesondere gegen pilzliche Schädlinge besitzen.

Überraschenderweise zeigen die erfindungsgemäßen 2-Aminomethyltetrahydrofurane der allgemeinen Formel (I) eine bessere fungizide Wirksamkeit als die aus dem Stand der Technik bekannten Aminomethyltetrahydrofurane, wie beispielsweise das 2-(4-Chlorphenyl)-5-(3,5-dimethylpiperidin-1-yl-methyl)-tetrahydrofuran, welches chemisch und wirkungsmäßig naheliegende Verbindungen sind.

Die erfindungsgemäßen Aminomethyltetrahydrofurane sind durch die Formel (I) allgemein definiert. Bevorzugt sind Verbindungen der Formel (I), bei welchen

$R^1$ für jeweils gegebenenfalls ein- bis dreifach, gleich oder verschieden substituiertes Phenyl steht, wobei als Substituenten infrage kommen: Fluor, Chlor, Brom, Iod, Cyano, Nitro, Methyl, Ethyl, n-, i-Propyl, n-, i-, s-, t-Butyl, n-, i-Pentyl, n-, i-Hexyl, Methoxy, Ethoxy, t-Butoxy, Trifluormethyl, Trifluormethylthio, Trifluormethoxy, Methoximinomethyl, Dimethylamino und Diethylamino; ferner für jeweils gegebenenfalls ein- bis dreifach, gleich oder verschieden substituiertes α-Naphthyl oder β-Naphthyl steht, wobei als Substituenten jeweils infrage kommen Fluor, Chlor, Brom, Iod, Methyl, Ethyl, n-, i-Propyl, n-, i-, s-, t-Butyl, Methoxy und Ethoxy; weiterhin für jeweils gegebenenfalls ein- bis dreifach, gleich oder verschieden substituiertes Cyclopentyl oder Cyclohexyl steht, wobei als Substituenten jeweils infrage kommen Methyl, Ethyl, n-, i-Propyl, n-, i-, s-, t-Butyl, n-, i-Pentyl, Methoxy, Ethoxy, t-Butoxy, Trifluormethyl und Difluormethoxy; ferner für gegebenenfalls ein- bis dreifach, gleich oder verschieden substituiertes Tetrahydronaphthyl steht, wobei als Substituenten infrage kommen: Fluor, Chlor Brom, Methyl, Ethyl, n-, i-Propyl, n-, i-, s-, t-Butyl, Methoxy und Ethoxy; weiterhin für gegebenenfalls ein- bis dreifach, gleich oder verschieden substituiertes Decahydronaphthyl steht, wobei als Substituenten infrage kommen: Methyl, Ethyl, n-, i-Propyl, n-, i-, s-, t-Butyl, Methoxy und Ethoxy;

oder für jeweils gegebenenfalls ein- bis dreifach, gleich oder verschieden substituiertes 2-, 3- oder 4-Pyridyl und 2- oder 3-Thienyl steht, wobei als Substituenten jeweils infrage kommen: Fluor, Chlor, Brom, Methyl, Ethyl, n-, i-Propyl, n-, i-, s- und t-Butyl;

$R^2$ für Wasserstoff oder Methyl steht und

$R^3$ und $R^4$ unabhängig voneinander jeweils für Wasserstoff, Methyl, Ethyl, n- oder i-Propyl, n-, i-, s- oder t-Butyl, n- oder i-Pentyl, n-oder i-Hexyl, n-oder i-Heptyl, n- oder i-Octyl, Allyl, n- oder i-Butenyl, n- oder i-Pentenyl, Propargyl, n- oder i-Butinyl, Hydroxyethyl, Hydroxypropyl, Methoxyethyl, Ethoxyethyl, Propoxyethyl, Butoxyethyl, Methoxypropyl, Ethoxypropyl, Propoxypropyl, Butoxypropyl, Hydroxyethoxyethyl, Dimethoxyethyl, Dimethoxypropyl, Diethoxyethyl, Dioxolanylmethyl, Dioxolanylethyl, Oxolanylmethyl, Oxolanylethyl, Dioxanylmethyl, Dioxanylethyl, für jeweils gegebenenfalls ein- bis fünffach; gleich oder verschieden durch Fluor, Chlor, Brom, Methyl, Ethyl, n- oder i-Propyl, n-, i-, s- und/oder t-Butyl substituiertes Cyclopropyl, Cyclopropylmethyl, Cyclopropylethyl, Cyclopropylpropyl, Cyclopentyl, Cyclopentylmethyl, Cyclohexyl oder Cyclohexylmethyl oder für jeweils gegebenenfalls ein- bis dreifach, gleich oder verschieden substituiertes Phenyl, Benzyl oder Phenylethyl stehen, wobei als Substituenten jeweils infrage kommen: Fluor, Chlor, Brom, Cyano, Nitro, Methyl, Ethyl, n-oder i-Propyl, n-, i-, s- oder t-Butyl, Methoxy, Ethoxy, Methylthio, Trifluormethoxy, Trifluormethylthio, Methoxycarbonyl, oder Methoximinomethyl;

oder

$R^3$ und $R^4$ gemeinsam mit dem Stickstoffatom, an welches sie gebunden sind, für einen gegebenenfalls ein- bis dreifach, gleich oder verschieden substituierten Heterocyclus der Formel

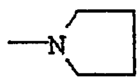 ; 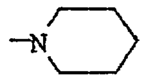 ; 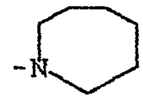 ; 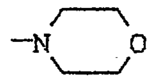

oder 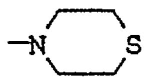

stehen, wobei als Substituenten infrage kommen: Methyl, Ethyl oder Hydroxymethyl.

Im einzelnen seien außer den bei den Herstellungsbeispielen genannten Verbindungen die folgenden 2-Aminomethyltetrahydrofurane der allgemeinen Formel (I) genannt:

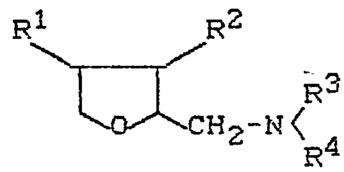 (I)

| $R^1$ | $R^2$ | $-N{<}^{R^3}_{R^4}$ |
|---|---|---|
| 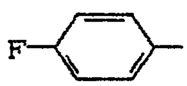 | H | 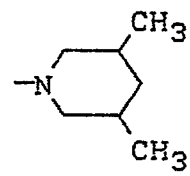 |
| 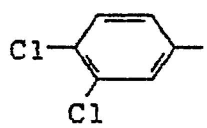 | H | 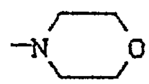 |
| 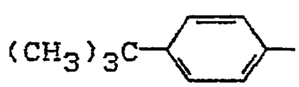 | H | 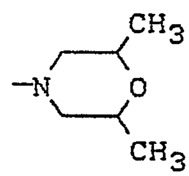 |
| 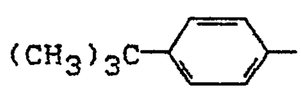 | H | $-NH-(CH_2)_3-OCH_3$ |

| $R^1$ | $R^2$ | 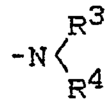 |
|---|---|---|
| 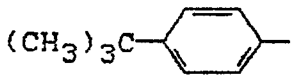 | H | $-NH-(CH_2)_3-OC_2H_5$ |
| 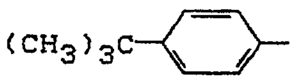 | H | 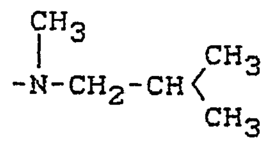 |
| 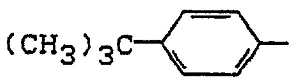 | H | 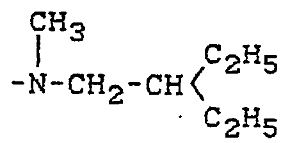 |
| 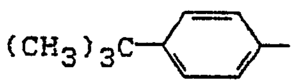 | H | 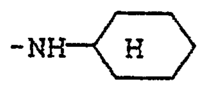 |
| 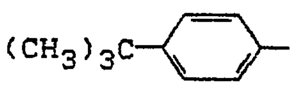 | H | 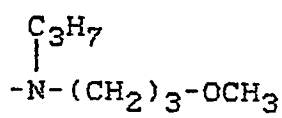 |
| 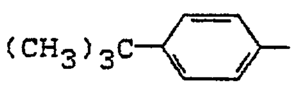 | H | $-NH-(CH_2)_2-O-(CH_2)_2-OH$ |
| 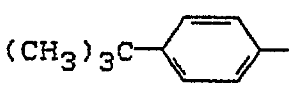 | H | 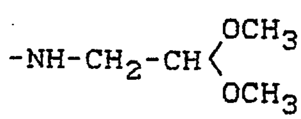 |
| 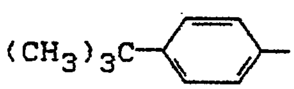 | H | 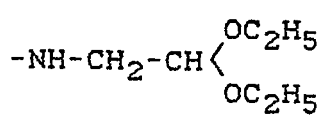 |

| $R^1$ | $R^2$ | 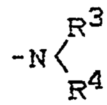 |
|---|---|---|
| 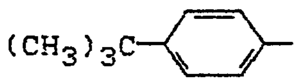 | H | 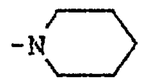 |
| 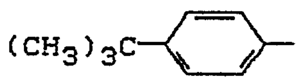 | H | 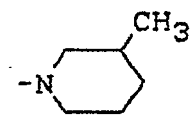 |
| 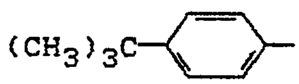 | H | 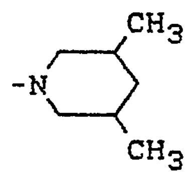 |
| 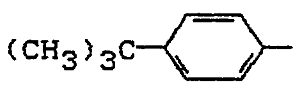 | CH3 | 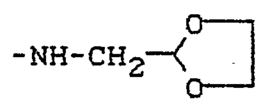 |
| 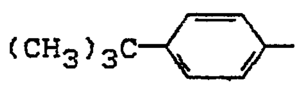 | $CH_3$ | 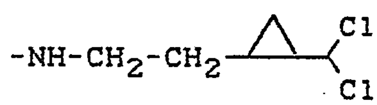 |
| 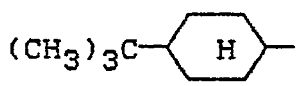 | $CH_3$ | $-NH-C_3H_7$ |
| 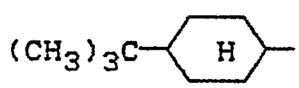 | H | 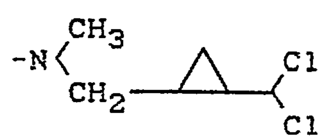 |
| 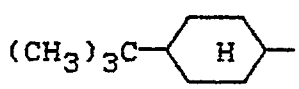 | H | 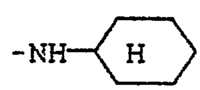 |

| $R^1$ | $R^2$ | $-N<^{R^3}_{R^4}$ |
|---|---|---|
| 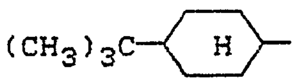 | H | 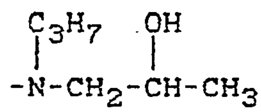 |
| 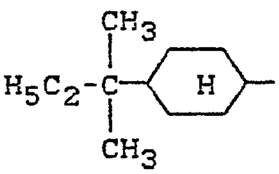 | H | 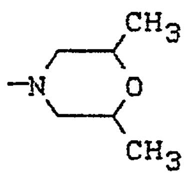 |
| 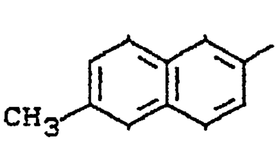 | H | 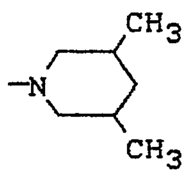 |
| 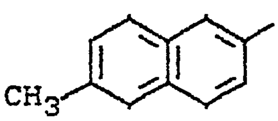 | H | $-NH-(CH_2)_3-OC_3H_7$ |
| 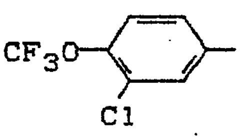 | H | $-NH-(CH_2)_3-OC_2H_5$ |
| 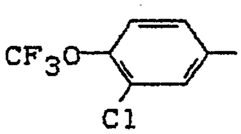 | $CH_3$ | 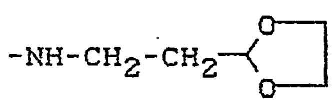 |
| 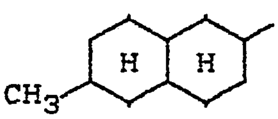 | H | 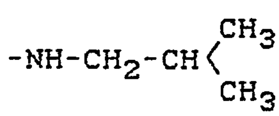 |

| $R^1$ | $R^2$ | 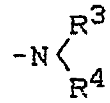 |
|---|---|---|
| 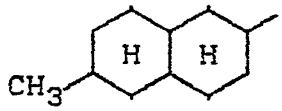 | $CH_3$ | 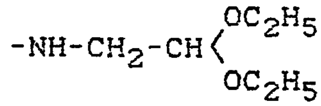 |
| 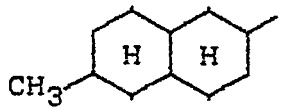 | H | $-NH-(CH_2)_3-OCH_3$ |
| 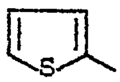 | $CH_3$ | $-NH(CH_2)_2-OCH_3$ |
| 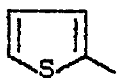 | H | $-NH-(CH_2)_2-O-(CH_2)_2-OH$ |
| 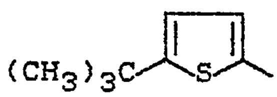 | H | 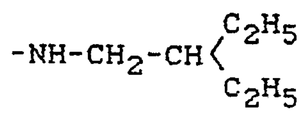 |
| 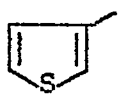 | $CH_3$ | 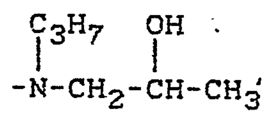 |
| 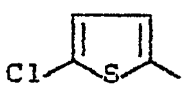 | H | 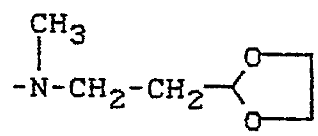 |
| 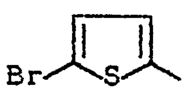 | H | $-NH-C_4H_9$ |

| $R^1$ | $R^2$ | 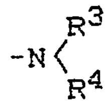 |
|---|---|---|
| 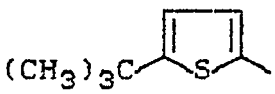 | H | 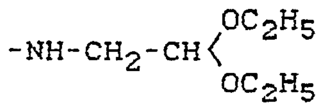 |
| 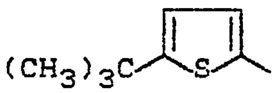 | H | 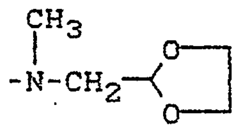 |
| 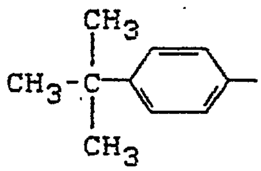 | H | 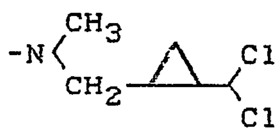 |
| 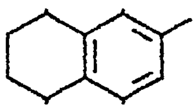 | H | 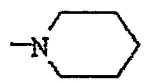 |
| 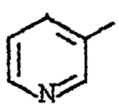 | H | 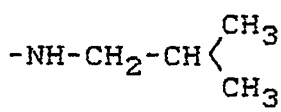 |
| 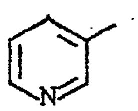 | $CH_3$ | 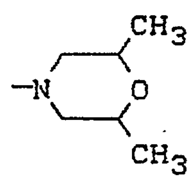 |
| 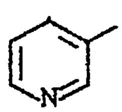 | H | 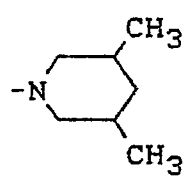 |

| $R^1$ | $R^2$ | 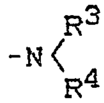 |
|---|---|---|
| 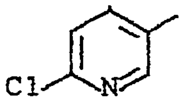 | H | 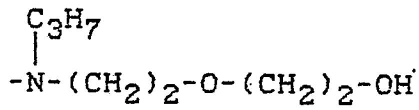 |
| 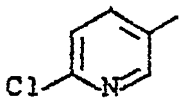 | H | 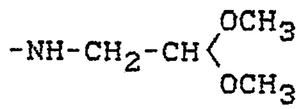 |
| 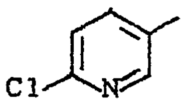 | H | $-NH-C_3H_7-n$ |
| 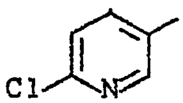 | H | 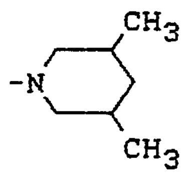 |
| 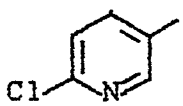 | H | 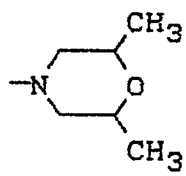 |
| 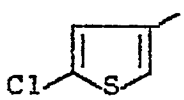 | H | 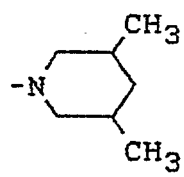 |
| 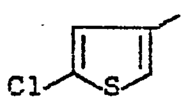 | H | 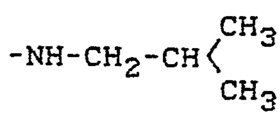 |

| $R^1$ | $R^2$ | $-N\genfrac{}{}{0pt}{}{R^3}{R^4}$ |
|---|---|---|
| 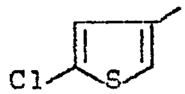 | H | $-NH-(CH_2)_3-OC_2H_5$ |
| 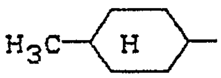 | H | 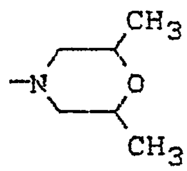 |
| 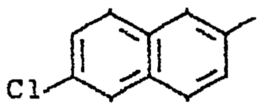 | H | 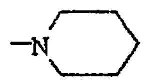 |
| 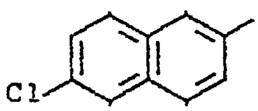 | H | 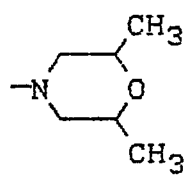 |
| 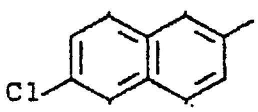 | H | $-NH-CH_2-CH\genfrac{}{}{0pt}{}{CH_3}{CH_3}$ |

Verwendet man beispielsweise 2-Brommethyl-4-cyclohexyltetrahydrofuran und Piperidin als Ausgangsstoffe, so läßt sich der Reaktionsablauf des erfindungsgemäßen Verfahrens (a) durch das folgende Formelschema darstellen:

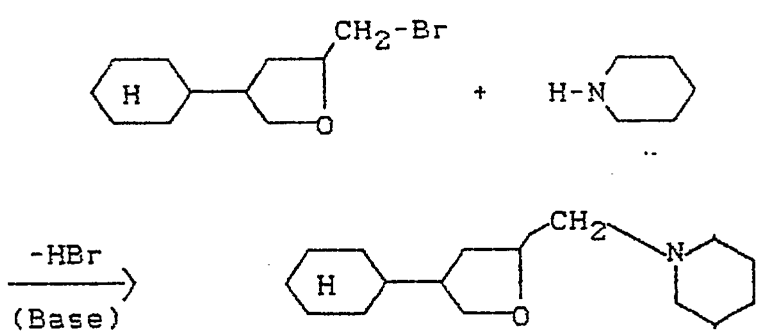


Verwendet man beispielsweise 2-Cyclohexylaminomethyl-4-[4-tert.-butylcyclohexyl]-tetrahydrofuran und Allylbromid als Ausgangsstoffe, so läßt sich der Reaktionsablauf des erfindungsgemäßen Verfahrens (b) durch das folgende Formelschema darstellen:

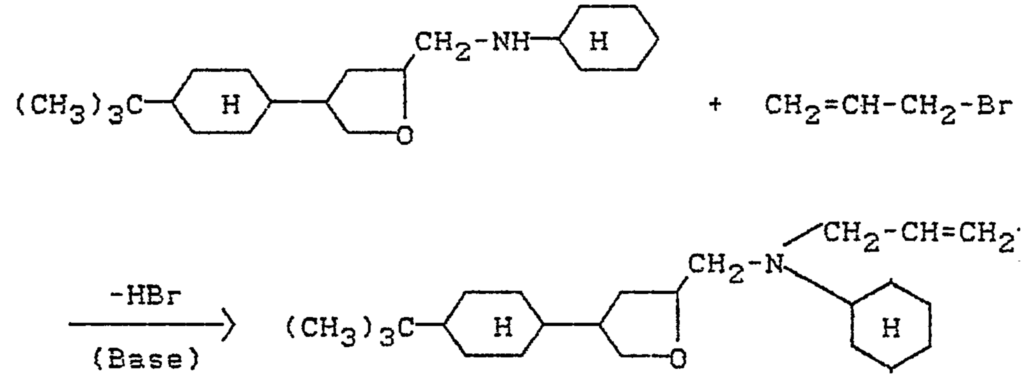


Verwendet man beispielsweise 2-(Morpholin-4-yl-methyl)-4-(4-tert.-butyl-phenyl)-tetrahydrofuran als Ausgangsstoff und Wasserstoff als Hydriermittel, so läßt sich der Reaktionsablauf des erfindungsgemäßen Verfahren (c) durch das folgende Formelschema darstellen:

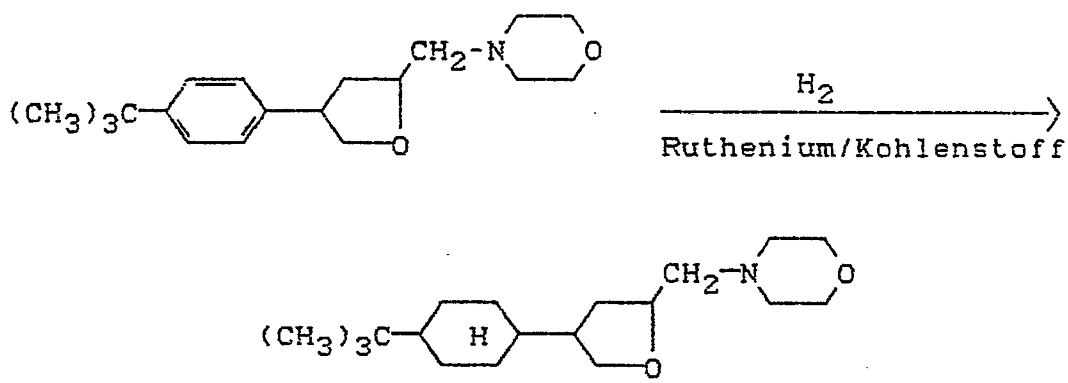


Die zur Durchführung des erfindungsgemäßen Verfahrens (a) als Ausgangsstoffe benötigen substituierten Tetrahydrofurane sind durch die Formel (II) allgemein definiert. In dieser Formel (II) stehen $R^1$ und $R^2$ vorzugsweise für diejenigen Reste, die bereits im Zusammenhang mit der Beschreibung der erfindungsgemäßen Stoffe der Formel (I) als bevorzugt für diese Substituenten genannt wurden.

$X^1$ steht vorzugsweise für Halogen, insbesondere für Chlor oder Brom oder für jeweils gegebenenfalls substituiertes Alkylsulfonyloxy oder Arylsulfonyloxy, wie beispielsweise Methansulfonyloxy oder p-Toluolsulfonyloxy,

Die substituierten Tetrahydrofurane der Formel (II) sind bekannt (vgl. z. B. EP-OS 68 331 und J. Org. Chem. 49(9), 1557–59) bzw. können sie in allgemein bekannter Art und Weise erhalten werden, beispielsweise, indem man die allgemein bekannten Ester der Formel (V)

$$\begin{array}{l} R^1-CH-COOR^5 \\ \quad\;\; | \\ R^2-CH-CH=CH_2 \end{array} \qquad (V)$$

in welcher

$R^1$ und $R^2$ die oben angegebene Bedeutung haben und

$R^5$ für Alkyl oder gegebenenfalls substituiertes Aryl steht,

mit einem Reduktionsmittel, wie beispielsweise Lithiumaluminiumhydrid, gegebenenfalls in Gegenwart eines Verdünnungsmittel, wie beispielweise Diethylether, bei Temperaturen zwischen –20°C und +80°C umsetzt;

anschließend die so erhaltenen Carbinole der Formel (VI)

$$\begin{array}{l} R^1-CH-CH_2OH \\ \quad\;\; | \\ R^2-CH-CH=CH_2 \end{array} \qquad (VI)$$

in welcher

$R^1$ und $R^2$ die oben angegebene Bedeutung haben, entweder mit einem Halogenierungsmittel, wie beispielsweise elementarem Brom, in Gegenwart von Chinolin oder mit N-Bromsuccinimid, gegebenenfalls in

Gegenwart eines Verdünnungsmittels, wie beispielweise Chloroform, bei Temperaturen zwischen –20°C und +80°C umsetzt, oder nach allgemein üblichen Verfahren, wie beispielsweise durch Umsetzung mit Wasserstoffperoxid/Ameisensäure in eine Trihydroxyverbindung der Formel (VII),

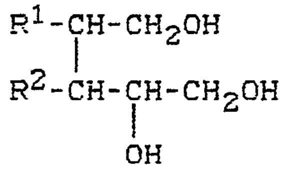 (VII)

in welcher

$R^1$ und $R^2$ die oben angegebene Bedeutung haben,

überführt und diese in situ oder nach Zwischenisolierung in allgemein bekannter Art und Weise mit wasserabspaltenden Reagenzien, wie beispielsweise Schwefelsäure, gegebenenfalls in Gegenwart eines Verdünnungsmittels, wie beispielsweise Toluol, bei Temperaturen zwischen –10°C und 120°C umsetzt;

und anschließend die so erhaltenen Tetrahydrofurane der Formel (VIII),

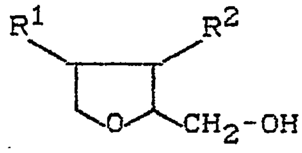 (VIII)

in welcher

$R^1$ und $R^2$ die oben angegebene Bedeutung haben,

mit üblichen Halogenierungsmitteln, wie beispielsweise Phosphortribromid, oder mit allgemein bekannten Sulfonsäurehalogeniden der Formel (IX),

$$R^6-SO_2-Hal \quad (IX)$$

in welcher

$R^6$ für jeweils gegebenenfalls substituiertes Alkyl oder Aryl, insbesondere für Methyl, Trifluormethyl oder p-Tolyl steht und

Hal für Halogen, insbesondere für Chlor, Brom oder Iod steht,

gegebenenfalls in Gegenwart eines Verdünnungsmittels, wie beispielsweise Dichlormethan, und gegebenenfalls in Gegenwart eines Säurebindemittels, wie beispielsweise Pyridin, bei Temperaturen zwischen 0°C und 120°C umsetzt.

Die zur Durchführung des erfindungsgemäßen Verfahrens (1) weiterhin als Ausgangsstoffe benötigten Amine sind durch die Formel (III) allgemein definiert. In dieser Formel (III) stehen $R^3$ und $R^4$ vorzugsweise für diejenigen Reste, die bereits im Zusammenhang mit der Beschreibung der erfindungsgemäßen Stoffe der Formel (I) als bevorzugt für diese Substituenten genannt wurden.

Die Amine der Formel (III) sind allgemein bekannte Verbindungen der organischen Chemie.

Die zur Durchführung des erfindungsgemäßen Verfahrens (b) als Ausgangsstoffe benötigten Aminomethyltetrahydrofurane sind durch die Formel (Ia) allgemein definiert.

In dieser Formel (Ia) stehen $R^1$, $R^2$ und $R^3$ vorzugsweise für diejenigen Reste, die bereits im Zusammenhang mit der Beschreibung der erfindungsgemäßen Stoffe der Formel (I) als bevorzugt für diese Substituenten genannt wurden.

Die Aminomethyltetrahydrofurane der Formel (Ia) sind erfindungsgemäße Verbindungen und erhältlich mit Hilfe des erfindungsgemäßen Verfahrens (a).

Die zur Durchführung des erfindungsgemäßen Verfahrens (b) weiterhin als Ausgangsstoffe benötigten Alkylierungsmittel sind durch die Formel (IV) allgemein definiert. In dieser Formel (IV) steht

$R^{4-1}$ vorzugsweise für Methyl, Ethyl, n- oder i-Propyl, n-, i-, s- oder t-Butyl, n- oder i-Pentyl, n- oder i-Hexyl, n- oder i-Heptyl, n- oder i-Octyl, Allyl, n- oder i-Butenyl, n- oder i-Pentenyl, Propargyl, n- oder i-Butinyl, Hydroxyethyl, Hydroxypropyl, Methoxyethyl, Ethoxyethyl, Propoxyethyl, Butoxyethyl, Methoxypropyl, Ethoxypropyl, Propoxypropyl, Butoxypropyl, Hydroxyethoxyethyl, Dimethoxyethyl, Dimethoxypropyl, Diethoxyethyl, Dioxolanylmethyl, Dioxolanylethyl, Dioxanylmethyl, Dioxanylethyl, jeweils für gegebenenfalls ein- bis fünffach, gleich oder verschieden durch Fluor, Chlor, Brom, Methyl, Ethyl, n- oder i-Propyl, n-, i-, s- und/oder t-Butyl substituiertes Cyclopropyl, Cyclopropylmethyl, Cyclopropylethyl, Cyclopropylpropyl, Cyclopentyl, Cyclopentylmethyl, Cyclohexyl oder Cyclohexylmethyl oder für jeweils gegebenenfalls ein- bis dreifach, gleich oder verschieden substituiertes Benzyl oder Phenylethyl, wobei als Substituenten jeweils infrage kommen: Fluor, Chlor, Brom, Cyano, Nitro, Methyl, Ethyl, n- oder i-Propyl, n-, i-, s- oder t-Butyl, Methoxy, Ethoxy, Methylthio, Trifluormethoxy, Trifluormethylthio, Methoxycarbonyl, Ethoxycarbonyl oder Methoximinomethyl,

$R^{4-1}$ steht ganz besonders bevorzugt für Methyl, Ethyl, n- oder i-Propyl, n-, i-, s- oder t-Butyl, n- oder i-Pentyl, n- oder i-Hexyl, Allyl, n- oder i-Butenyl, n- oder i-Pentenyl, Propargyl, n- oder i-Butinyl, Hydroxyethyl, Hydroxypropyl, Methoxyethyl, Methoxypropyl, Ethoxyethyl, Ethoxypropyl, Hydroxyethoxyethyl, Dimethoxyethyl, Diethoxyethyl, Dioxolanylmethyl, Dioxolanylethyl, Oxolanylmethyl, Oxolanylethyl, Dioxanylmethyl, Cyclopropylmethyl, Dichlorcyclopropylmethyl, Dimethylcyclopropylmethyl, Dichlordimethylcyclopropylmethyl, Cyclopentyl, Cylohexyl oder Cyclohexylmethyl

$X^2$ steht vorzugsweise für Halogen, insbesondere für Chlor, Brom oder Jod oder für jeweils gegebenenfalls substituiertes Alkylsulfonyloxy, Alkoxysulfonyloxy oder Arylsulfonyloxy, wie beispielsweise Methansulfonyloxy, Methoxysulfonyloxy oder p-Toluolsulfonyloxy.

Die Alkylierungsmittel der Formel (IV) sind allgemein bekannte Verbindungen der organischen Chemie bzw. in Analogie zu allgemein bekannten Verfahren erhältlich.

Die zur Durchführung des erfindungsgemäßen Verfahrens (c) als Ausgangsstoffe benötigten Aminomethyltetrahyrdrofurane sind durch die Formel (Ib) allgemein definiert.

Die Aminomethyltetrahydrofurane der Formel (Ib) sind erfindungsgemäße Stoffe und gemäß Verfahren (a) erhältlich.

Als Verdünnungsmittel zur Durchführung der erfindungsgemäßen Verfahren (a) und (b) kommen inerte organische Lösungsmittel oder wäßrige Systeme infrage. Hierzu gehören insbesondere aliphatische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl oder -diethylether, Ketone, wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester oder Sulfoxide, wie Dimethylsulfoxid.

Die erfindungsgemäßen Verfahren (a) und (b) können gegebenenfalls auch in einem Zweiphasensystem, wie beispielsweise Wasser/Toluol oder Wasser/Dichlormethan, gegebenenfalls in Gegenwart eines Phasentransferkatalysators durchgeführt werden. Als Beispiele für solche Katalysatoren seien genannt: Tetrabutylammoniumiodid, Tetrabutylammoniumbromid, Tributyl-methylphosphoniumbromid, Trimethyl-$C_{13}$/$C_{15}$-alkylammoniumchlorid, Dibenzyldimethyl-ammoniummethylsulfat, Dimethyl-$C_{12}$/$C_{14}$-alkylbenzylammoniumchlorid, Tetrabutylammoniumhydroxid, 15-Krone-5, 18-Krone-6, Triethylbenzylammoniumchlorid, Trimethylbenzylammoniumchlorid. Es ist auch möglich, die erfindungsgemäßen Verfahren (a) und (b) ohne Zusatz eines Lösungsmittels durchzuführen.

Als Säurebindemittel zur Durchführung der erfindungsgemäßen Verfahren (a) und (b) kommen alle üblicherweise verwendbaren anorganischen und organischen Basen infrage. Vorzugsweise verwendet man Alkalimetallhydroxide, -carbonate oder -hydrogencarbonate, wie beispielsweise Natriumhydroxid, Natriumcarbonat oder Natriumhydrogencarbonat oder auch tertiäre Amine, wie beispielweise Triethylamin, N,N-Dimethylanilin, Pyridin, 4-(N,N-Dimethylamino)-pyridin, Diazabicyclooctan (DABCO), Diazabicyclononen (DBN) oder Diazabicycloundecen (DBU).

Es ist auch möglich, die als Reaktionsteilnehmer verwendeten Amine der Formeln (III) bzw. (Ia) in entsprechendem Überschuß gleichzeitig als Säurebindemittel einzusetzen.

Die Reaktionstemperaturen können bei der Durchführung der erfindungsgemäßen Verfahren (a) und (b) in einem größeren Bereich variiert werden. Im allgemeinen arbeitet man bei Temperaturen zwischen –20°C und +180°C, vorzugsweise bei Temperaturen zwischen 20°C und +150°C.

Zur Durchführung des erfindungsgemäßen Verfahrens (a) setzt man pro Mol an substituiertem Tetrahydrofuran der Formel (II) im allgemeinen 1,0 bis 3,0 Mol vorzugsweise 1,0 bis 1,5 Mol an Amin der Formel (III) und gegebenenfalls 1,0 bis 3,0 Mol, vorzugsweise 1,0 bis 1,5 Mol an Säurebindemittel, sowie gegebenenfalls 0,1 bis 1,0 Mol an Phasentransferkatalysator ein.

Zur Durchführung des erfindungsgemäßen Verfahrens (b) setzt man pro Mol an Aminomethyltetrahydrofuran der Formel (Ia) im allgemeinen 1,0 bis 5,0 Mol, vorzugsweise 1,0 bis 2,0 Mol an Alkylierungsmittel der Formel (IV) und 1,0 bis 5,0 Mol, vorzugsweise 1,0 bis 2,0 Mol an Säurebindemittel, sowie gegebenenfalls 0,1 bis 1,0 Mol an Phasentransferkatalysator ein. Die Reaktionsdurchführung, Aufarbeitung und Isolierung der Reaktionsprodukte der Formel (I) erfolgt in beiden Fällen nach üblichen Methoden.

Als Verdünnungsmittel kommen bei der Durchführung des erfindungsgemäßen Verfahrens (c) alle unter Hydrierungsbedingungen inerten Solventien in Betracht. Vorzugsweise verwendbar sind Kohlenwasserstoffe, wie Petrolether, Pentan, Hexan, Heptan, Cyclohexan, Alkohole, wie Methanol, Ethanol, n-Propanol und i-Propanol oder Ether, wie Diethylether, Dioxan, Tetrahydrofuran oder Ethylenglykoldimethyl- oder -diethylether. Es kann jedoch auch ohne Verdünnungsmittel gearbeitet werden.

Als Hydrierungsmittel kommen bei der Durchführung des erfindungsgemäßen Verfahrens (c) diejenigen Verbindungen infrage, die Aromaten hydrieren können. Vorzugsweise verwendet man molekularen Wasserstoff.

Als Hydrierungskatalysatoren können bei dem erfindungsgemäßen Verfahren (c) alle üblicherweise für derartige Reaktionen verwendbaren Katalysatoren eingesetzt werden. Vorzugsweise verwendet man Raney-Nickel oder Edelmetallkatalysatoren, wie Palladium, Ruthenium, Palladiumoxid, Platin oder Platinoxid, gegebenenfalls auf einem geeigneten Trägerstoff, wie z.B. Kohle.

Die Reaktionstemperaturen können bei dem erfindungsgemäßen Verfahren (c) in einem größeren Bereich variiert werden. Im allgemeinen arbeitet man bei Temperaturen zwischen 50°C und 250°C, vorzugsweise bei Temperaturen zwischen 80°C und 200°C.

Das erfindungsgemäße Verfahren (c) kann bei Normaldruck, aber auch bei erhöhtem Druck durchgeführt werden. Bevorzugt wird unter erhöhtem Druck in Gegenwart von Wasserstoff gearbeitet. Im allgemeinen arbeitet man bei Druckverhältnissen zwischen 5 und 300 atm, vorzugsweise zwischen 10 und 200 atm.

Bei der Durchführung des erfindungsgemäßen Verfahrens (c) setzt man auf 1 Mol Aminomethyltetrahydrofuran der Formel (Ib) im allgemeinen 3,0 bis 30 Mol Hydrierungsmittel und 0,001 bis 0,1 Mol, vorzugsweise 0,01 bis 0,1 Mol Katalysator ein.

Bei der Durchführung des erfindungsgemäßen Verfahrens (c) geht man im allgemeinen so vor, daß man die Verbindungen der Formel (Ib) in Gegenwart eines Verdünnungsmittels bei der jeweils gewünschten Temperatur mit Wasserstoff im Autoklaven umsetzt. Die Aufarbeitung geschieht nach üblichen Methoden.

Zur Herstellung von Säureadditionssalzen der Verbindungen der Formel (I) kommen vorzugsweise folgende Säuren infrage: Halogenwasserstoffsäuren, wie z.B. Chlorwasserstoffsäure und Bromwasserstoffsäure, insbesondere Chlorwasserstoffsäure, ferner Phosphorsäure, Salpetersäure, Schwefelsäure, mono-, bi- und trifunktionelle Carbonsäuren und Hydroxycarbonsäuren, wie z.B. Essigsäure, Maleinsäure, Bernsteinsäure, Fumarsäure, Weinsäure, Zitronensäure, Salicylsäure, Sorbinsäure, Milchsäure, sowie Sulfonsäuren, wie z.B. p-Toluolsulfonsäure und 1,5-Naphthalindisulfonsäure sowie Saccharin.

Die Säureadditionssalze der Verbindungen der Formel (I) können in einfacher Weise nach üblichen Salzbildungsmethoden, wie z.B. durch Lösen einer Verbindung der Formel (I) in einem geeigneten inerten Lösungsmittel und Hinzufügen der Säure, wie z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Die erfindungsgemäßen Wirkstoffe weisen eine starke Wirkung gegen Schädlinge auf und können zur Bekämpfung von unerwünschten Schadorganismen praktisch eingesetzt werden. Die Wirkstoffe sind für den Gebrauch u.a. als Pflanzenschutzmittel insbesondere als Fungizide geeignet.

Fungizide Mittel im Pflanzenschutz werden eingesetzt zur Bekämpfung von Plasmodiophoromycetes, Oomycetes, Chyrtridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt:

Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea (Konidienform: Drechslera, Syn: Helminthosporium);
Cochliobolus-Arten, wie beispielsweise Cochliobolus sativus (Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Die neuen Wirkstoffe können mit besonders gutem Erfolg zur Bekämpfung von Getreidekrankheiten, wie beispielsweise Erysiphe graminis, Septoria nodorum, Cochliobolus sativus und Pyrenophora teres

eingesetzt werden. Darüber hinaus zeigen die Verbindungen der Formel (I) gute Wirkung gegen Reiskrankheiten, wie Pyricularia oryzae, sowie eine breite fungizide in-vitro-Wirksamkeit.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemäßen Wirkstoffe können in den Formulierungen in Mischung mit anderen bekannten Wirkstoffen vorliegen wie Fungizide, Insektizide, Akarizide und Herbizide sowie in Mischungen mit Düngemitteln und Wachstumsregulatoren.

Die Wirkstoffe können als solche, in Form ihrer Formulierungen oder den daraus bereiteten Anwendungsformen wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%., vorzugsweise zwischen 0,5 und 0,001%.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, vorzugsweise von 0,0001 bis 0,02% am Wirkungsort erforderlich.

Herstellungsbeispiele:

Beispiel 1:

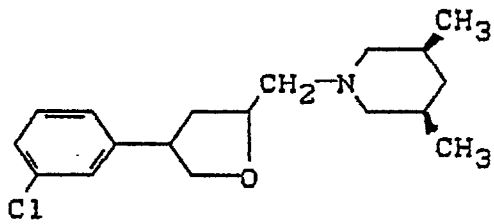

Eine Mischung aus 20 g (0,073 Mol) 2-Brommethyl-4-(3-chlorphenyl)-tetrahydrofuran und 17 g (0,147 Mol) cis-3,5-Dimethylpiperidin wird 16 Stunden bei 120°C Badtemperatur gerührt, abgekühlt und in einer Mischung aus Diethylether und Wasser aufgenommen; die organische Phase wird abgetrennt, über Natriumsulfat getrocknet, im Vakuum eingeengt und der Rückstand über Kieselgel chromatographiert.

Man erhält 8,3 g (37% der Theorie) 2-(3,5-cis-Dimethylpiperidin-1-yl-methyl)-4-(3-chlorphenyl)-tetrahydrofuran vom Brechungsindex $n_D^{20} = 1{,}5548$.

Herstellung der Ausgangsverbindung

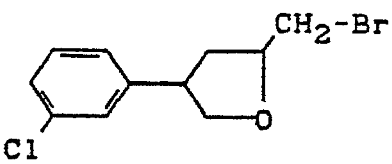

Zu einer Lösung von 51 g (0,26 Mol) 2-(3-Chlorphenyl)-2-allyl-ethanol in 400 ml absolutem Chloroform gibt man unter Rühren portionsweise 46,3 g (0, 6 Mol) N-Bromsuccinimid, wobei sich die Reaktionsmischung bis auf 40°C erwärmen soll. Nach beendeter Zugabe rührt man weitere 16 Stunden bei Raumtemperatur, wäscht zweimal mit Wasser, trocknet über Natriumsulfat und entfernt das Lösungsmittel im Vakuum.

Man erhält 47 g (66% der Theorie) 2-Brommethyl-4-(3-chlorphenyl)-tetrahydrofuran, das ohne Reinigung und Isolierung direkt weiter umgesetzt wird.

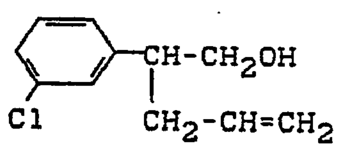

Zu einer Lösung von 76 g (0,32 Mol) 2-(3-Chlorphenyl)-2-allyl-essigsäureethylester in 250 ml absolutem Ether gibt man portionsweise 6 g (0,15 Mol) Lithiumaluminiumhydrid, rührt anschließend 16 Stunden bei Rückflußtemperatur und hydrolysiert dann mit konzentrierter Ammoniumchlorid-Lösung. Die organische Phase wird abgetrennt, über Natriumsulfat getrocknet und im Vakuum vom Lösungsmittel befreit.

Man erhält 51 g (81% der Theorie) 2-(3-Chlorphenyl)-2-allyl-ethanol (IR: 3200–3300), das ohne weitere Reinigung und Isolierung direkt weiter umgesetzt wird.

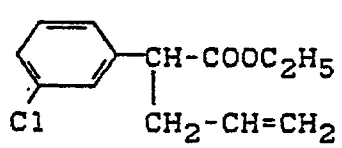

Zu einer Lösung von 114 g (0,597 Mol) 2-Allyl-3-chlorphenylacetonitril in 150 ml Ethanol tropft man 150 g konzentrierte Schwefelsäure und rührt nach beendeter Zugabe 16 Stunden bei 110°C Badtemperatur. Nach dem Abkühlen gießt man auf Eis und extrahiert mit Petrolether. Die organische Phase wird mit Natriumhydrogencarbonatlösung neutral gewaschen, über Natriumsulfat getrocknet und im Vakuum vom Lösungsmittel befreit. Der Rückstand wird im Vakuum destilliert.

Man erhält 84 g (59% der Theorie) 2-Allyl-3-chlorphenylessigsäureethylester vom Siedepunkt 120°C /0,1 Torr.

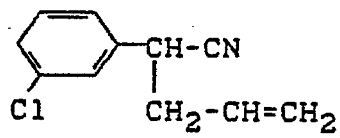

Zu einer auf 0°C gekühlten Lösung von 151 g (1 Mol) 3-Chlorbenzylcyanid in 500 ml Dimethylformamid gibt man portionsweise 28,7 g (1 Mol) Natriumhydrid (80%), wobei die Temperatur durch Kühlung zwischen 0°C und 10°C gehalten wird. Nach beendeter Wasserstoffentwicklung tropft man bei einer Temperatur von 30°C 122 g (1 Mol) Allylbromid zu und rührt anschließend noch 16 Stunden bei 45°C. Die Reaktionsmischung wird mit Wasser verdünnt und mit Ether extrahiert. Die organische Phase wird über Natriumsulfat getrocknet, vom Lösungsmittel befreit und der Rückstand im Vakuum destilliert.

Man erhält 114 g (60% der Theorie) 2-Allyl-3-chlorphenylacetonitril vom Siedepunkt 91–95°C/0,1 Torr.

In entsprechender Weise und gemäß den allgemeinen Angaben zur Herstellung erhält man die folgenden 2-Aminomethyltetrahydrofurane der allgemeinen Formel (I):

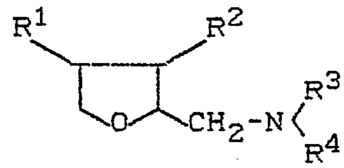

(I)

| Bsp. Nr. | $R^1$ | $R^2$ | $-N{<}^{R^3}_{R^4}$ | Physikalische Eigenschaften [$n^{20}$ = Brechungsindex bzw. $^1$HNMR] |
|---|---|---|---|---|
| 2 | 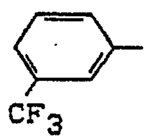 | H | 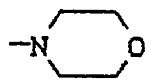 | 1,5169 |
| 3 | 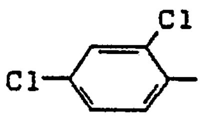 | H | 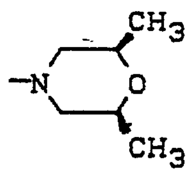 | 1,5430 |
| 4 | 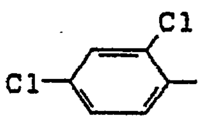 | H | 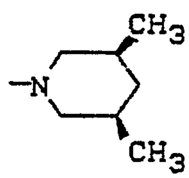 | 1,5456 |
| 5 | 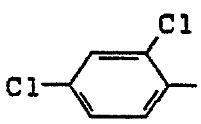 | H | 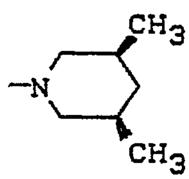 | 4,5-4,8(m)<br>4,1-4,4(m)<br>3,75-3,9(m)<br>3,5 -3,7(m) |
| | | | x 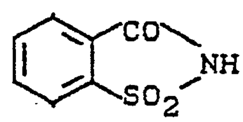 | |
| 6 | 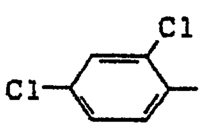 | H | 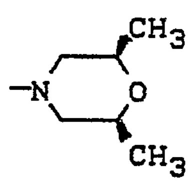 | 4,55-4,8(m)<br>4,1 -4,3(m)<br>3,55-4,0(m) |
| | | | x 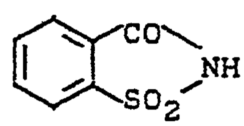 | |

| Bsp. Nr. | $R^1$ | $R^2$ | $-N{<}^{R^3}_{R^4}$ | Physikalische Eigenschaften [$n^{20}$ = Brechungsindex bzw. $^1HNMR$] |
|---|---|---|---|---|
| 7 | 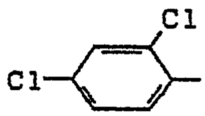 | $CH_3$ | 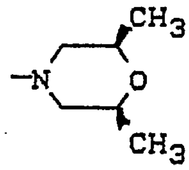 | 1,5503 |
| 8 | 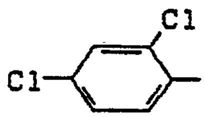 | $CH_3$ | 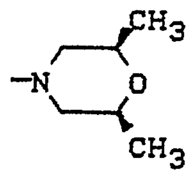<br>x 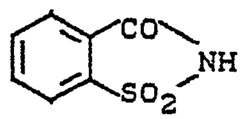 | Fp. 37-42° C |
| 9 | 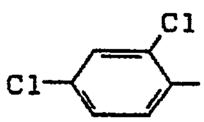 | $CH_3$ | 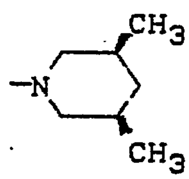 | 1,5464 |
| 10 | 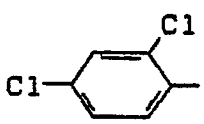 | $CH_3$ | 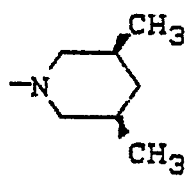<br>x 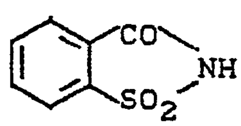 | Fp. 33-37° C |
| 11 | 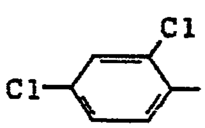 | $CH_3$ | $-NH-CH_2-CH{<}^{CH_3}_{CH_3}$ | 1,5096 |

| Bsp. Nr. | $R^1$ | $R^2$ | $-N{<}^{R^3}_{R^4}$ | Physikalische Eigenschaften [$n^{20}$ = Brechungsindex bzw. $^1HNMR$] |
|---|---|---|---|---|
| 12 | 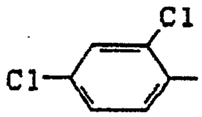 | $CH_3$ | 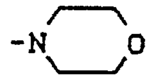 | 1,5496 |
| 13 | 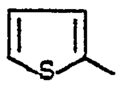 | H | 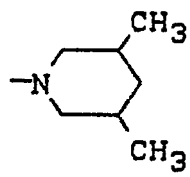 | 1,574 |
| 14 | 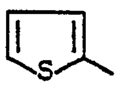 | H | 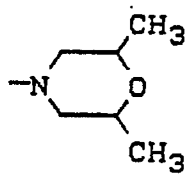 | 1,5259 |
| 15 | 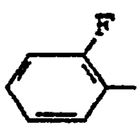 | H | $-NH-(CH_2)_3-O-(CH_2)_3-CH_3$ | 1.4989 |
| 16 | 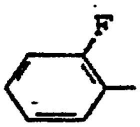 | H | 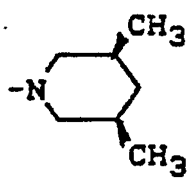 | 1.5151 |
| 17 | 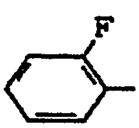 | H | 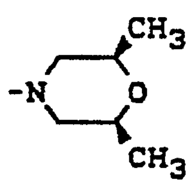 | 1.5130 |
| 18 | 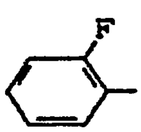 | $CH_3$ | $-NH-(CH_2)_3-O-(CH_2)_3-CH_3$ | 1.4961 |

| Bsp. Nr. | $R^1$ | $R^2$ | $-N{<}^{R^3}_{R^4}$ | Physikalische Eigenschaften [$n^{20}$ = Brechungsindex bzw. $^1$HNMR] |
|---|---|---|---|---|
| 19 | 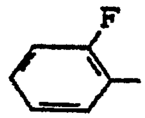 | $CH_3$ | 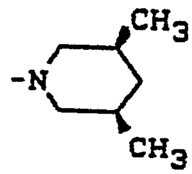 | 1.5120 |
| 20 | 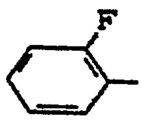 | $CH_3$ | 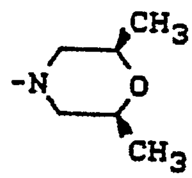 | 1.5089 |
| 21 | 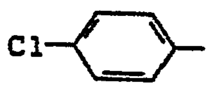 | $CH_3$ | 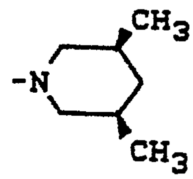 | 1.5261 |
| 22 | 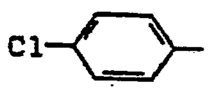 | $CH_3$ | 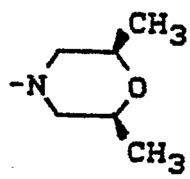 | 1.5275 |
| 23 | 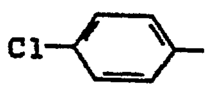 | $CH_3$ | 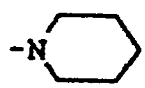 | 1.5348 |
| 24 | 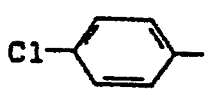 | $CH_3$ | $-NH-(CH_2)_3-O-C_2H_5$ | 1.5993 |
| 25 | 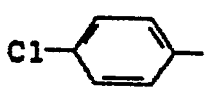 | $CH_3$ | $-NH-CH_2-CH(CH_3)_2$ | 1.5168 |

| Bsp. Nr. | $R^1$ | $R^2$ | $-N\langle^{R^3}_{R^4}$ | Physikalische Eigenschaften [$n^{20}$ = Brechungsindex bzw. $^1$HNMR] |
|---|---|---|---|---|
| 26 | 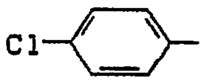 | $CH_3$ | $-NH-(CH_2)_5-CH_3$ | 1.5179 |
| 27 | 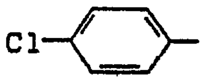 | $CH_3$ | 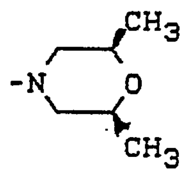 | |
| | | | x 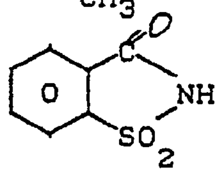 | Fp. 32° C |
| 28 | 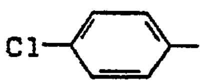 | $CH_3$ | 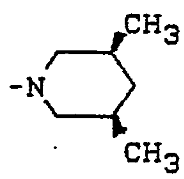 | |
| | | | x 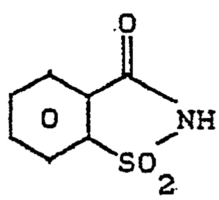 | Fp. 35° C |
| 29 | 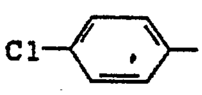 | $CH_3$ | 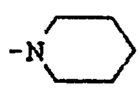 | |
| | | | x 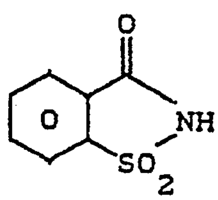 | Fp.33-35° C |

| Bsp. Nr. | $R^1$ | $R^2$ | $-N\langle^{R^3}_{R^4}$ | Physikalische Eigenschaften [$n^{20}$ = Brechungsindex bzw. $^1$HNMR] |
|---|---|---|---|---|
| 30 | 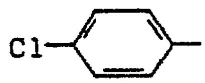 | $CH_3$ | $-NH-(CH_2)_5-CH_3$ | |
| | | | x 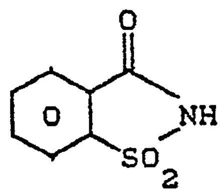 | 1.5369 |
| 31 | 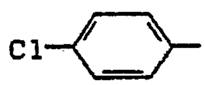 | $CH_3$ | $-NH-(CH_2)_3-OC_2H_5$ | |
| | | | x 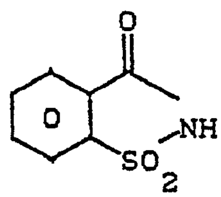 | 1.5348 |
| 32 | 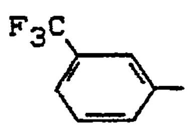 | H | 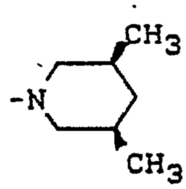 | 1.4826 |
| 33 | 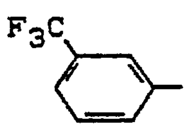 | H | 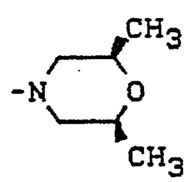 | 1.4869 |
| 34 | 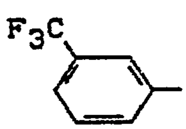 | H | $-NH-CH_2-CH(CH_3)_2$ | 1.4731 |

| Bsp. Nr. | $R^1$ | $R^2$ | $-N\langle^{R^3}_{R^4}$ | Eigenschaften [$n^{20}$ = Brechungsindex bzw. $^1$HNMR] |
|---|---|---|---|---|
| 35 | 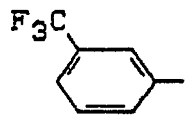 | H | $-NH-(CH_2)_3-OC_2H_5$ | 1.4769 |
| 36 | 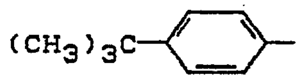 | H | 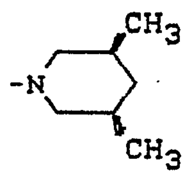 | 1.5104 |
| 37 | 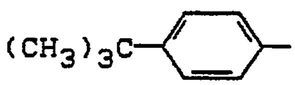 | H | 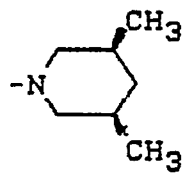 | 1.5099 |
| 38 | 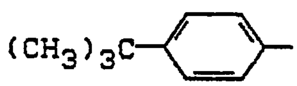 | H | 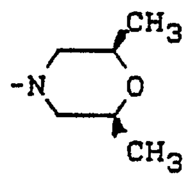 | |
| | | | x 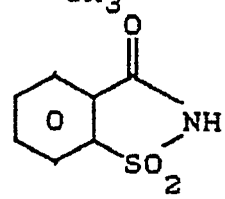 | Fp. 41-43° C |
| 39 | 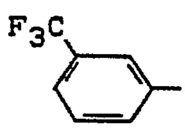 | H | 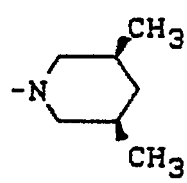 | |
| | | | x 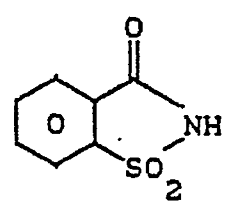 | 1.5302 |

| Bsp. Nr. | $R^1$ | $R^2$ | $-N\langle^{R^3}_{R^4}$ | Physikalische Eigenschaften [$n^{20}$ = Brechungsindex bzw. $^1$HNMR] |
|---|---|---|---|---|
| 40 | 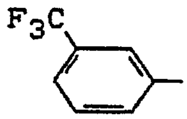 | H | 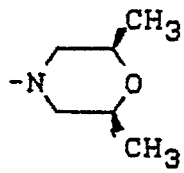 | |
| | | | x 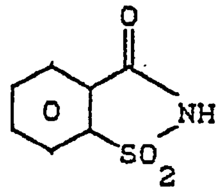 | Fp. 31-34° C |
| 41 | 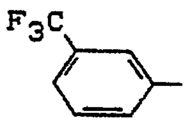 | $CH_3$ | 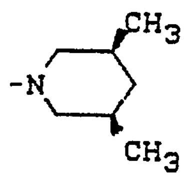 | 1.4794 |
| 42 | 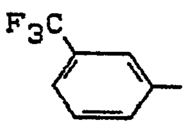 | H | $-NH(CH_2)_3-OC_2H_5$ | |
| | | | x 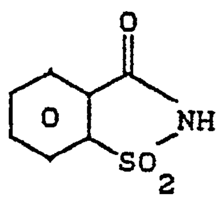 | Fp. ~30° C |
| 43 | 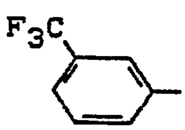 | $CH_3$ | 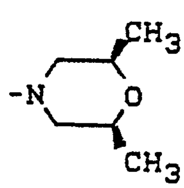 | 1.4861 |
| 44 | 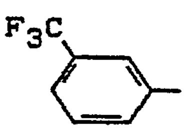 | $CH_3$ | $-NH-(CH_2)_3-OC_2H_5$ | 1.4779 |

| Bsp. Nr. | $R^1$ | $R^2$ | -N $R^3$ $R^4$ | Physikalische Eigenschaften [$n^{20}$ = Brechungsindex bzw. $^1$HNMR] |
|---|---|---|---|---|
| 45 | 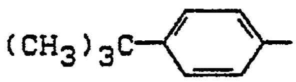 | $CH_3$ | 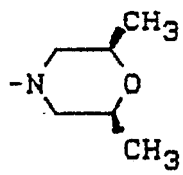 | 1.5140 |
| 46 | 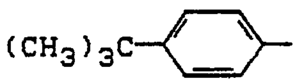 | $CH_3$ | 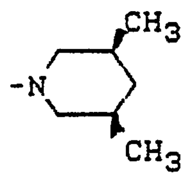 | 1.5155 |
| 47 | 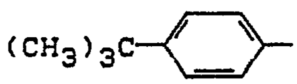 | $CH_3$ | $-NH-(CH_2)_3-OC_2H_5$ | 1.5050 |
| 48 | 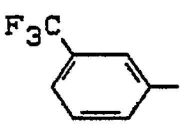 | $CH_3$ | $-NH-(CH_2)_3-OC_2H_5$ | |
| | | | x 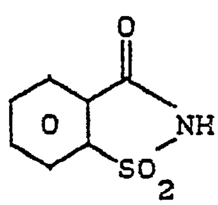 | 1.5299 |
| 49 | 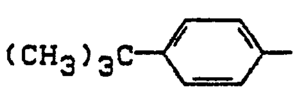 | $CH_3$ | 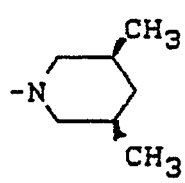 | |
| | | | x 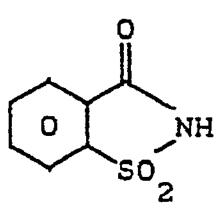 | 1.5340 |

| Bsp. Nr. | $R^1$ | $R^2$ | $-N{<}^{R^3}_{R^4}$ | Physikalische Eigenschaften $[n^{20}$ = Brechungs-index bzw. $^1$HNMR] |
|---|---|---|---|---|
| 50 | 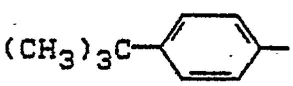 | $CH_3$ | 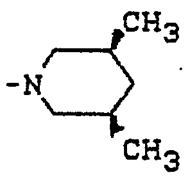 | |
| | | | x 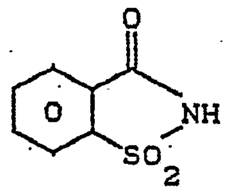 | 1.5480 |
| 51 | 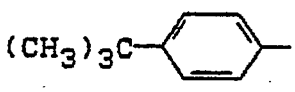 | H | 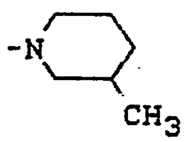 | 1.5176 |
| 52 | 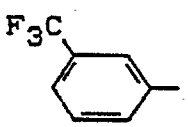 | $CH_3$ | 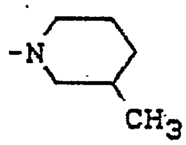 | 1.4889 |
| 53 | 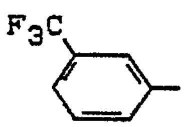 | $CH_3$ | 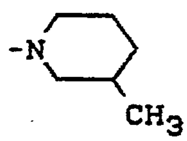 | |
| | | | x 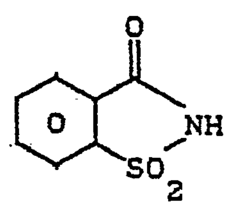 | Fp. 36° C |

| Bsp. Nr. | $R^1$ | $R^2$ | $-N<^{R^3}_{R^4}$ | Physikalische Eigenschaften [$n^{20}$ = Brechungsindex bzw. $^1HNMR$] |
|---|---|---|---|---|
| 54 | 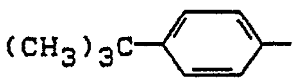 | H | 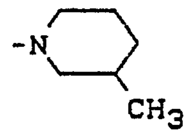 | |
| | | | x 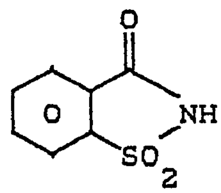 | Fp. 34° C |
| 55 | 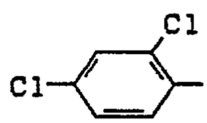 | H | 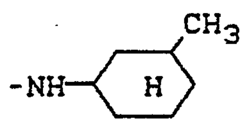 | 1.5381 |
| 56 | 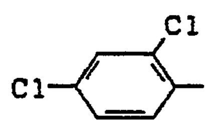 | H | 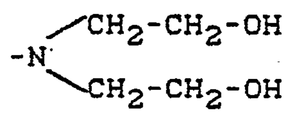 | 1.5574 |
| 57 | 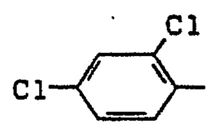 | H | 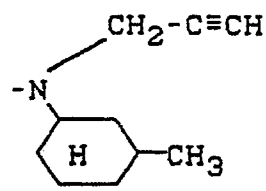 | 1.5474 |
| 58 | 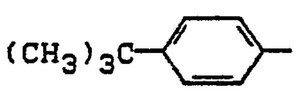 | H | 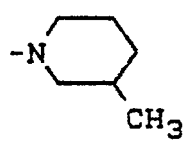 | |
| | | | x 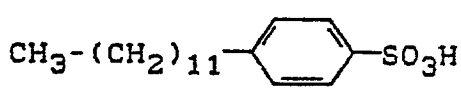 | |

| Bsp. Nr. | $R^1$ | $R^2$ | $-N{<}^{R^3}_{R^4}$ | Physikalische Eigenschaften [$n^{20}$ = Brechungsindex bzw. $^1HNMR$] |
|---|---|---|---|---|
| 59 | 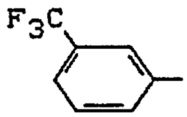 | $CH_3$ | 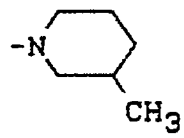 | |
| | | | x 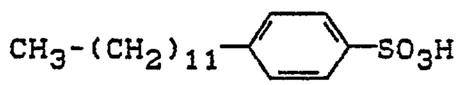 | |
| 60 | 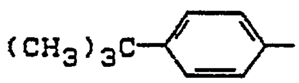 | H | 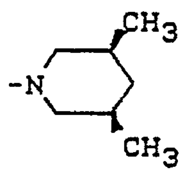 | |
| | | | x 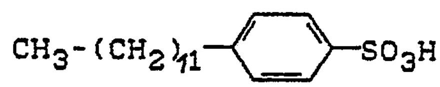 | |
| 61 | 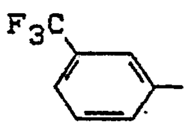 | H | 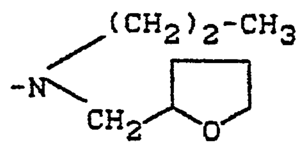 | 1.4815 |
| 62 | 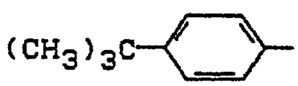 | H | 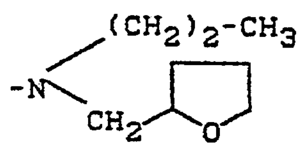 | 1.5106 |

| Bsp. Nr. | $R^1$ | $R^2$ | 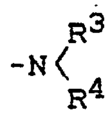 | Physikalische Eigenschaften [$n^{20}$ = Brechungsindex bzw. $^1$HNMR] |
|---|---|---|---|---|
| 63 | 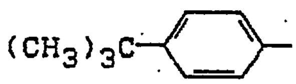 | H | 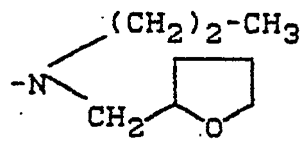 | |
| | | | x 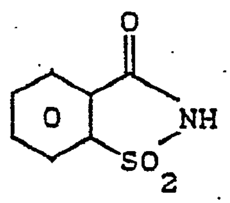 | Fp. 36° C |
| 64 | 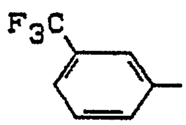 | $CH_3$ | 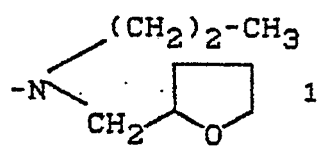 | 1.4822 |
| 65 | 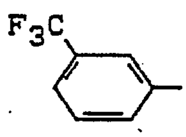 | H | 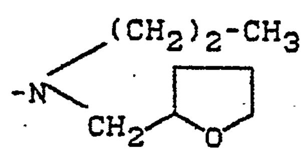 | |
| | | | x 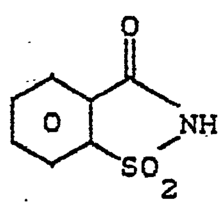 | Fp. 33-36° C |
| 66 | 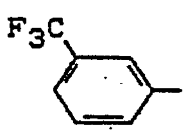 | $CH_3$ | 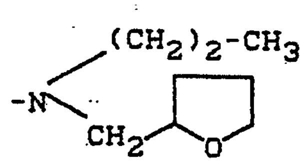 | |
| | | | x 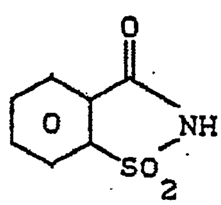 | Fp. 34° C |

| Bsp. Nr. | $R^1$ | $R^2$ | $-N{<}^{R^3}_{R^4}$ | Physikalische Eigenschaften [$n^{20}$ = Brechungsindex bzw. $^1$HNMR] |
|---|---|---|---|---|
| 67 | 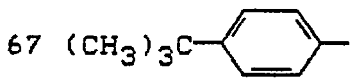 | $CH_3$ | 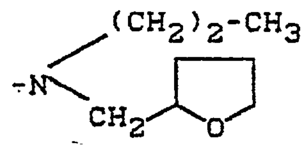 | 1.5121 |
| 68 | 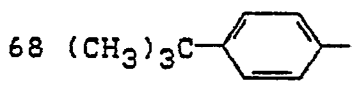 | $CH_3$ | 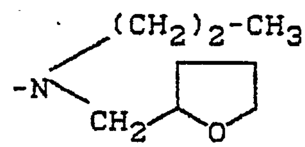 x 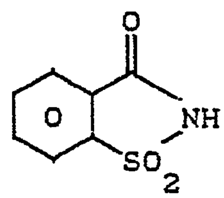 | 1.5415 |
| 69 | 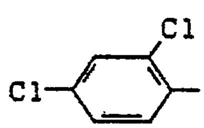 | H | 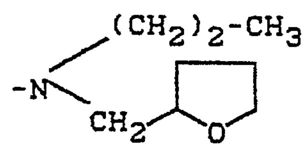 | 1.5405 |

## Anwendungsbeispiele

In den nachfolgenden Anwendungsbeispielen wird die nachstehend aufgeführte Verbindung als Vergleichssubstanz verwendet:

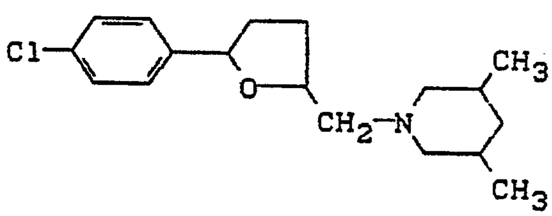

(A)

2-(4-Chlorphenyl)-5-(3,5-dimethylpiperidin-1-yl-methyl-tetrahydrofuran (vgl. DE-OS 3 413 996).

## Beispiel A

Erysiphe-Test (Gerste) / protektiv
Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp.hordei bestäubt.

Die Pflanzen werden in einem Gewächshaus bei einer Temperatur von ca. 20°C und einer relativen Luftfeuchtigkeit von ca. 80% aufgestellt, um die Entwicklung von Mehltaupusteln zu begünstigen.

7 Tage nach der Inokulation erfolgt die Auswertung.

Eine deutliche Überlegenheit in der Wirksamkeit gegenüber dem Stand der Technik zeigen bei diesem Test z.B. die Verbindungen gemäß den Herstellungsbeispielen: 3, 4, 9 und 10.

Beispiel B

Pyricularia-Test (Reis) / protektiv
Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100%. rel. Luftfeuchtigkeit und 25°C aufgestellt.

4 Tage nach der Inokulation erfolgt die Auswertung des Krankheitsbefalls.

Eine deutliche Überlegenheit in der Wirksamkeit gegenüber dem Stand der Technik zeigen in diesem Test z.B. die Verbindungen gemäß den Herstellungsbeispielen: 5, 6 und 8.

Beispiel C

Pyricularia-Test (Reis) / systemisch
Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf systemische Eigenschaften werden 40 ml der Wirkstoffzubereitung auf Einheitserde gegossen, in der junge Reispflanzen angezogen wurden. 7 Tage nach der Behandlung werden die Pflanzen mit einer wäßrigen Sporensuspension von Pyricularia oryzae inokuliert. Danach verbleiben die Pflanzen in einem Gewächshaus bei einer Temperatur von 25°C und einer rel. Luftfeuchtigkeit von 100% bis zur Auswertung.

4 Tage nach der Inokulation erfolgt die Auswertung des Krankheitsbefalls.

Eine deutliche Überlegenheit in der Wirksamkeit gegenüber dem Stand der Technik zeigen in diesem Test z.B. die Verbindungen gemäß den Herstellungsbeispielen: 5, 6 und 8.

## Patentansprüche

1. 2-Aminomethyltetrahydrofurane der allgemeinen Formel (I)

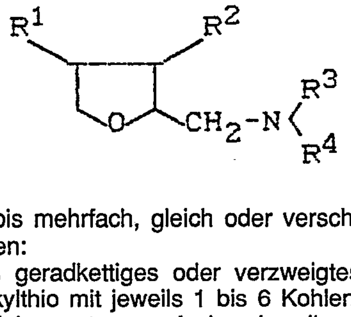

(I)

in welcher

$R^1$ für gegebenenfalls einfach bis mehrfach, gleich oder verschieden substituiertes Phenyl steht, wobei als Substituenten infrage kommen:
Halogen, Cyano, Nitro, jeweils geradkettiges oder verzweigtes Alkyl, Alkoxy, Alkylthio, Halogenalkyl, Halogenalkoxy oder Halogenalkylthio mit jeweils 1 bis 6 Kohlenstoffatomen und gegebenenfalls 1 bis 9 gleichen oder verschiedenen Halogenatomen, Amino, jeweils geradkettiges oder verzweigtes Alkylamino, Dialkylamino oder Alkoximinoalkyl mit jeweils 1 bis 4 Kohlenstoffatomen in den einzelnen Alkylteilen; ferner für jeweils gegebenenfalls einfach bis mehrfach, gleich oder verschiedenen substituiertes α-

Naphthyl oder β-Naphthyl steht, wobei als Substituenten jeweils infrage kommen: Halogen, jeweils geradkettiges oder verzweigtes Alkyl oder Alkoxy mit jeweils 1 bis 4 Kohlenstoffatomen;
weiterhin für gegebenenfalls einfach bis mehrfach, gleich oder verschieden substituiertes Cycloalkyl mit 3 bis 7 Kohlenstoffatomen steht, wobei als Substituenten infrage kommen: jeweils geradkettiges oder verzweigtes Alkyl, Alkoxy, Halogenalkyl oder Halogenalkoxy mit jeweils 1 bis 6 Kohlenstoffatomen und gegebenenfalls 1 bis 9 gleichen oder verschiedenen Halogenatomen;
ferner für jeweils gegebenenfalls einfach bis mehrfach, gleich oder verschieden substituiertes Tetrahydronaphthyl oder Decahydronaphthyl steht, wobei als Substituenten für die hydrierten Ringe infrage kommen: geradkettiges oder verzweigtes Alkyl und Alkoxy mit jeweils 1 bis 6 Kohlenstoffatomen, sowie zusätzlich für die aromatischen Ringe Halogen;
weiterhin für einen gegebenenfalls einfach bis mehrfach, gleich oder verschieden durch Alkyl mit 1 bis 6 Kohlenstoffatomen und/oder Halogen substituierten 5- oder 6-gliedrigen Heteroarylrest mit 1 oder 2 Heteroatomen,
$R^2$ für Wasserstoff oder Methyl steht und
$R^3$ und $R^4$ unabhängig voneinander jeweils für Wasserstoff; für jeweils geradkettiges oder verzweigtes Alkyl mit 1 bis 12 Kohlenstoffatomen, Alkenyl mit 3 bis 8 Kohlenstoffatomen, Alkinyl mit 3 bis 8 Kohlenstoffatomen, Hydroxyalkyl mit 2 bis 6 Kohlenstoffatomen, Alkoxyalkyl oder Dialkoxyalkyl mit jeweils 1 bis 8 Kohlenstoffatomen oder Hydroxyalkoxyalkyl mit 2 bis 8 Kohlenstoffatomen in den einzelnen Alkylteilen; für jeweils geradkettiges oder verzweigtes Dioxolanylalkyl, Oxolanylalkyl oder Dioxanylalkyl mit jeweils 1 bis 4 Kohlenstoffatomen im Alkylteil oder für jeweils gegebenenfalls im Cycloalkylteil einfach bis mehrfach, gleich oder verschieden substituiertes Cycloalkyl oder Cycloalkylalkyl mit jeweils 3 bis 7 Kohlenstoffatomen im Cycloalkylteil und gegebenenfalls 1 bis 4 Kohlenstoffatomen im geradkettigen oder verzweigten Alkylteil stehen, wobei als Substituenten jeweils infrage kommen:
Halogen, jeweils geradkettiges oder verzweigtes Alkyl, Alkoxy, Halogenalkyl oder Halogenalkoxy mit jeweils 1 bis 4 Kohlenstoffatomen und gegebenenfalls 1 bis 9 gleichen oder verschiedenen Halogenatomen; außerdem für jeweils gegebenenfalls einfach bis mehrfach, gleich oder verschieden im Arylteil substituiertes Arylalkyl, Arylalkenyl oder Aryl mit jeweils 6 bis 10 Kohlenstoffatomen im Arylteil und gegbenenfalls mit bis zu 6 Kohlenstoffatomen im geradkettigen oder verzweigten Alkyl- bzw. Alkenylteil stehen, wobei als Arylsubstituenten jeweils infrage kommen:
Halogen, Cyano, Nitro, jeweils geradkettiges oder verzweigtes Alkyl, Alkoxy, Alkylthio, Halogenalkyl, Halogenalkoxy, Halogenalkylthio oder Alkoximinoalkyl mit jeweils 1 bis 4 Kohlenstoffatomen in den einzelnen Alkylteilen und gegebenenfalls 1 bis 9 gleichen oder verschiedenen Halogenatomen, oder
$R^3$ und $R^4$ gemeinsam mit dem Stickstoffatom, an welches sie gebunden sind, für einen gegebenenfalls substituierten gesättigten Heterocyclus stehen, der gegebenenfalls weitere Heteroatome enthalten kann, wobei als Substituenten infrage kommen: jeweils geradkettiges oder verzweigtes Alkyl oder Hydroxyalkyl mit jeweils 1 bis 4 Kohlenstoffatomen, deren Säureadditionssalze, deren geometrische und optische Isomere und deren Isomerengemische.

2. 2-Aminomethyltetrahydrofurane gemäß Anspruch 1, wobei in der Formel (I)
$R^1$ für jeweils gegebenenfalls ein- bis dreifach, gleich oder verschieden substituiertes Phenyl steht, wobei als Substituenten infrage kommen: Fluor, Chlor, Brom, Iod, Cyano, Nitro, Methyl, Ethyl, n-, i-Propyl, n-, i-, s-, t-Butyl, n-, i-Pentyl, n-, i-Hexyl, Methoxy, Ethoxy, t-Butoxy, Trifluormethyl, Trifluormethylthio, Trifluormethoxy, Methoximinomethyl, Dimethylamino und Diethylamino;
ferner für jeweils gegebenenfalls ein- bis dreifach, gleich oder verschieden substituiertes α-Naphthyl oder β-Naphthyl steht, wobei als Substituenten jeweils infrage kommen: Fluor, Chlor, Brom, Iod, Methyl, Ethyl, n-, i-Propyl, n-, i-, s-, t-Butyl, Methoxy und Ethoxy;
weiterhin für jeweils gegebenenfalls ein- bis dreifach, gleich oder verschieden substituiertes Cyclopentyl oder Cyclohexyl steht, wobei als Substituenten jeweils infrage kommen: Methyl, Ethyl, n-, i-Propyl, n-, i-, s-, t-Butyl, n-, i-Pentyl, Methoxy, Ethoxy, t-Butoxy, Trifluormethyl und Difluormethoxy;
ferner für gegebenenfalls ein- bis dreifach, gleich oder verschieden substituiertes Tetrahydronaphthyl steht, wobei als Substituenten infrage kommen: Fluor, Chlor, Brom, Methyl, Ethyl, n-, i-Propyl, n-, i-, s-, t-Butyl, Methoxy und Ethoxy;
weiterhin für gegebenenfalls ein- bis dreifach, gleich oder verschieden substituiertes Decahydronaphthyl steht, wobei als Substituenten infrage kommen: Methyl, Ethyl, n-, i-Propyl, n-, i-, s-, t-Butyl, Methoxy und Ethoxy;
oder für jeweils gegebenenfalls ein- bis dreifach, gleich oder verschieden substituiertes 2-, 3- oder 4-Pyridyl und 2- oder 3-Thienyl steht, wobei als Substituenten jeweils infrage kommen: Fluor, Chlor, Brom, Methyl, Ethyl, n-, i-Propyl, n-, i-, s- und t-Butyl;
$R^2$ für Wasserstoff oder Methyl steht und
$R^3$ und $R^4$ unabhängig voneinander jeweils für Wasserstoff, Methyl, Ethyl, n- oder i-Propyl, n-, i-, s- oder t-Butyl, n- oder i-Pentyl, n- oder i-Hexyl, n- oder i-Heptyl, n- oder i-Octyl, Allyl, n- oder i-Butenyl, n- oder i-Pentenyl, Propargyl, n- oder i-Butinyl, Hydroxyethyl, Hydroxypropyl, Methoxyethyl, Ethoxyethyl, Propoxyethyl, Butoxyethyl, Methoxypropyl, Ethoxypropyl, Propoxypropyl, Butoxypropyl, Hydroxyethoxyethyl, Dimethoxyethyl, Dimethoxypropyl, Diethoxyethyl, Dioxolanylmethyl, Dioxolanylethyl, Oxolanylmethyl, Oxolanylethyl, Dioxanylmethyl, Dioxanylethyl, für jeweils gegebenenfalls ein- bis fünffach, gleich oder verschieden durch Fluor, Chlor, Brom, Methyl, Ethyl, n- oder i-Propyl, n-, i-, s-und t-Butyl substituiertes Cyclopropyl, Cyclopropylmethyl, Cyclopropylethyl, Cyclopropylpropyl, Cyclopentyl, Cyclopentylmethyl, Cyclohexyl oder Cyclohexylmethyl oder für jeweils gegebenenfalls ein- bis dreifach, gleich oder verschieden substituiertes Phenyl, Benzyl oder Phenylethyl stehen, wobei als Substituenten jeweils infrage kommen: Fluor, Chlor, Brom, Cyano, Nitro, Methyl, Ethyl, n- oder i-Propyl, n-, i-, s- oder t-Butyl, Methoxy, Ethoxy, Methylthio, Trifluormethoxy, Trifluormethylthio, Methoxycarbonyl, oder Methoximinomethyl deren Säureadditionssalze, deren geometrische und optische Isomere und deren Isomerengemische.

3. 2-Aminomethyltetrahydrofurane gemäß Anspruch 1, wobei in der Formel (I)
$R^1$ für jeweils gegebenenfalls ein- bis dreifach, gleich oder verschieden substituiertes Phenyl steht, wobei als Substituenten infrage kommen: Fluor, Chlor, Brom, Iod, Cyano, Nitro, Methyl, Ethyl, n- i-Propyl, n-, i-, s-, t-Butyl, n-, i-Pentyl, n-, i-Hexyl, Methoxy, Ethoxy, t-Butoxy, Trifluormethyl, Trifluormethylthio, Trifluormethoxy, Methoximinomethyl, Dimethylamino und Diethylamino;
ferner für jeweils gegebenenfalls ein- bis dreifach, gleich oder verschieden substituiertes α-Naphthyl oder β-Naphthyl steht, wobei als Substituenten jeweils infrage kommen: Fluor, Chlor, Brom, Iod, Methyl, Ethyl, n-, i-Propyl, n-, i-, s-, t-Butyl, Methoxy und Ethoxy;
weiterhin für jeweils gegebenenfalls ein- bis dreifach, gleich oder verschieden substituiertes Cyclopentyl oder Cyclohexyl steht, wobei als Substituenten jeweils infrage kommen: Methyl, Ethyl, n-, i-Propyl, n-, i-, s-, t-Butyl, n-, i-Pentyl, Methoxy, Ethoxy, t-Butoxy, Trifluormethyl und Difluormethoxy;
ferner für gegebenenfalls ein- bis dreifach, gleich oder verschieden substituiertes Tetrahydronaphthyl steht, wobei als Substituenten infrage kommen: Fluor, Chlor, Brom, Methyl, Ethyl, n-, i-Propyl, n-, i-, s-, t-Butyl, Methoxy und Ethoxy;
weiterhin für gegebenenfalls ein- bis dreifach, gleich oder verschieden substituiertes Decahydronaphthyl steht, wobei als Substituenten infrage kommen: Methyl, Ethyl, n-, i-Propyl, n-, i-, s-, t-Butyl, Methoxy und Ethoxy;
oder für jeweils gegebenenfalls ein- bis dreifach, gleich oder verschieden substituiertes 2-, 3- oder 4-Pyridyl oder 2- oder 3-Thienyl steht, wobei als Substituenten jeweils infrage kommen: Fluor, Chlor, Brom, Methyl, Ethyl, n-, i-Propyl, n-, i-, s- und t-Buty
$R^2$ für Wasserstoff oder Methyl steht und
$R^3$ und $R^4$ gemeinsam mit dem Stickstoffatom, an welches sie gebunden sind, für einen gegebenenfalls ein- bis dreifach, gleich oder verschieden substituierten Heterocyclus der Formel

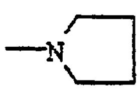 ; 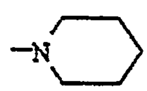 ; 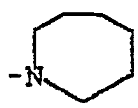 ; 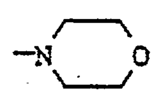

oder 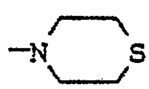

stehen, wobei als Substituenten infrage kommen: Methyl, Ethyl oder Hydroxymethyl, deren Säureadditionssalze, deren geometrische und optische Isomere und deren Isomerengemische.

4. Verfahren zur Herstellung von 2-Aminomethyltetrahydrofuranen der allgemeinen Formel (I),

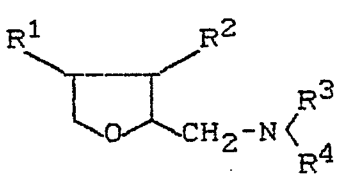 (I)

in welcher
$R^1$ für gegebenenfalls einfach bis mehrfach, gleich oder verschieden substituiertes Phenyl steht, wobei als Substituenten infrage kommen:
Halogen, Cyano, Nitro, jeweils geradkettiges oder verzweigtes Alkyl, Alkoxy, Alkylthio, Halogenalkyl, Halogenalkoxy oder Halogenalkylthio mit jeweils 1 bis 6 Kohlenstoffatomen und gegebenenfalls 1 bis 9 gleichen oder verschiedenen Halogenatomen, Amino, jeweils geradkettiges oder verzweigtes Alkylamino, Dialkylamino oder Alkoximinoalkyl mit jeweils 1 bis 4 Kohlenstoffatomen in den einzelnen Alkylteilen;
ferner für jeweils gegebenenfalls einfach bis mehrfach, gleich oder verschieden substituiertes α-Naphthyl oder β-Naphthyl steht, wobei als Substituenten jeweils infrage kommen:
Halogen, jeweils geradkettiges oder verzweigtes Alkyl oder Alkoxy mit jeweils 1 bis 4 Kohlenstoffatomen;
weiterhin für gegebenenfalls einfach bis mehrfach, gleich oder verschieden substituiertes Cycloalkyl mit 3 bis 7 Kohlenstoffatomen steht, wobei als Substituenten infrage kommen:
jeweils geradkettiges oder verzweigtes Alkyl, Alkoxy, Halogenalkyl oder Halogenalkoxy mit jeweils 1 bis

6 Kohlenstoffatomen und gegebenenfalls 1 bis 9 gleichen oder verschiedenen Halogenatomen; ferner für jeweils gegebenenfalls einfach bis mehrfach, gleich oder verschieden substituiertes Tetrahydronaphthyl oder Decahydronaphthyl steht, wobei als Substituenten für die hydrierten Ringe infrage kommen: geradkettiges oder verzweigtes Alkyl und Alkoxy mit jeweils 1 bis 6 Kohlenstoffatomen, sowie zusätzlich für die aromatischen Ringe Halogen; weiterhin für einen gegebenenfalls einfach bis mehrfach, gleich oder verschieden durch Alkyl mit 1 bis 6 Kohlenstoffatomen und/oder Halogen substituierten 5- oder 6-gliedrigen Heteroarylrest mit 1 oder 2 Heteroatomen,

$R^2$ für Wasserstoff oder Methyl steht und

$R^3$ und $R^4$ unabhängig voneinander jeweils für Wasserstoff; für jeweils geradkettiges oder verzweigtes Alkyl mit 1 bis 12 Kohlenstoffatomen, Alkenyl mit 3 bis 8 Kohlenstoffatomen, Alkinyl mit 3 bis 8 Kohlenstoffatomen, Hydroxyalkyl mit 2 bis 6 Kohlenstoffatomen, Alkoxyalkyl oder Dialkoxyalkyl mit jeweils 1 bis 8 Kohlenstoffatomen oder Hydroxyalkoxyalkyl mit 2 bis 8 Kohlenstoffatomen in den einzelnen Alkylteilen; für jeweils geradkettiges oder verzweigtes Dioxolanylalkyl, Oxolanylalkyl oder Dioxanylalkyl mit jeweils 1 bis 4 Kohlenstoffatomen im Alkylteil oder für jeweils gegebenenfalls im Cycloalkylteil einfach bis mehrfach, gleich oder verschieden substituiertes Cycloalkyl oder Cycloalkylalkyl mit jeweils 3 bis 7 Kohlenstoffatomen im Cycloalkylteil und gegebenenfalls 1 bis 4 Kohlenstoffatomen im geradkettigen oder verzweigten Alkylteil stehen, wobei als Substituenten jeweils infrage kommen: Halogen, jeweils geradkettiges oder verzweigtes Alkyl, Alkoxy, Halogenalkyl oder Halogenalkoxy mit jeweils 1 bis 4 Kohlenstoffatomen und gegebenenfalls 1 bis 9 gleichen oder verschiedenen Halogenatomen; außerdem für jeweils gegebenenfalls einfach bis mehrfach, gleich oder verschieden im Arylteil substituiertes Arylalkyl, Arylalkenyl oder Aryl mit jeweils 6 bis 10 Kohlenstoffatomen im Arylteil und gegebenenfalls mit bis zu 6 Kohlenstoffatomen im geradkettigen oder verzweigten Alkyl- bzw. Alkenylteil stehen, wobei als Arylsubstituenten jeweils infrage kommen: Halogen, Cyano, Nitro, jeweils geradkettiges oder verzweigtes Alkyl, Alkoxy, Alkylthio, Halogenalkyl, Halogenalkoxy, Halogenalkylthio oder Alkoximinoalkyl mit jeweils 1 bis 4 Kohlenstoffatomen in den einzelnen Alkylteilen und gegebenenfalls 1 bis 9 gleichen oder verschiedenen Halogenatomen, oder

$R^3$ und $R^4$ gemeinsam mit dem Stickstoffatom, an welches sie gebunden sind, für einen gegebenenfalls substituierten gesättigten Heterocyclus stehen, der gegebenenfalls weitere Heteroatome enthalten kann, wobei als Substituenten infrage kommen: jeweils geradkettiges oder verzweigtes Alkyl oder Hydroxyalkyl mit jeweils 1 bis 4 Kohlenstoffatomen, deren Säureadditionssalze, deren geometrische und optische Isomere und deren Isomerengemische, dadurch gekennzeichnet, daß man

(a) substituierte Tetrahydrofurane der Formel (II),

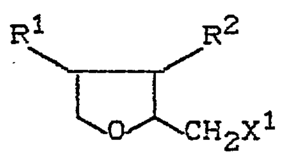

(II),

in welcher

$R^1$ und $R^2$ die oben angegebene Bedeutung haben und

$X^1$ für eine elektronenanziehende Abgangsgruppe steht,

mit Aminen der Formel (III),

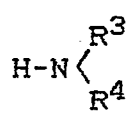

(III)

in welcher

$R^3$ und $R^4$ die oben angegebene Bedeutung haben,

gegebenenfalls in Gegenwart eines Verdünnungsmittels und gegebenenfalls in Gegenwart eines Säurebindemittels umsetzt; oder

(b) die nach Verfahren (a) und/oder (c) erhältlichen Aminomethyltetrahydrofurane der Formel (Ia),

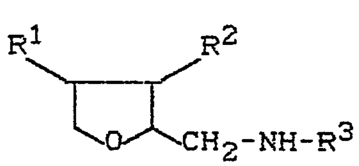

(Ia)

in welcher

$R^1$, $R^2$ und $R^3$ die oben angegebene Bedeutung haben,

mit Alkylierungsmitteln der Formel (IV),

$$R^{4-1}-X^2 \qquad (IV)$$

in welcher
$R^{4-1}$ für jeweils geradkettiges oder verzweigtes Alkyl mit 1 bis 12 Kohlenstoffatomen, Alkenyl mit 3 bis 8 Kohlenstoffatomen, Alkinyl mit 3 bis 8 Kohlenstoffatomen, Hydroxyalkyl mit 2 bis 6 Kohlenstoffatomen, Alkoxyalkyl, Dialkoxyalkyl oder Hydroxyalkoxyalkyl mit jeweils 1 bis 6 Kohlenstoffatomen in den einzelnen Alkylteilen, für Dioxolanylalkyl, Oxolanylalkyl oder Dioxanylalkyl mit jeweils 1 bis 4 Kohlenstoffatomen im geradkettigen oder verzweigten Alkylteil, für jeweils gegebenenfalls im Cycloalkylteil einfach bis mehrfach, gleich oder verschieden substituiertes Cycloalkyl oder Cycloalkylalkyl mit jeweils 3 bis 7 Kohlenstoffatomen im Cycloalkylteil und gegebenenfalls 1 bis 4 Kohlenstoffatomen im geradkettigen oder verzweigten Alkylteil, wobei als Substituenten jeweils infrage kommen: Halogen, jeweils geradkettiges oder verzweigtes Alkyl, Alkoxy, Halogenalkyl oder Halogenalkoxy mit jeweils 1 bis 4 Kohlenstoffatomen und gegebenenfalls 1 bis 9 gleichen oder verschiedenen Halogenatomen; außerdem für jeweils gegebenenfalls einfach bis mehrfach, gleich oder verschieden im Arylteil substituiertes Arylalkyl oder Arylalkenyl mit jeweils 6 bis 10 Kohlenstoffatomen im Arylteil und bis zu 6 Kohlenstoffatomen im geradkettigen oder verzweigten Alkyl- bzw. Alkenylteil, wobei als Arylsubstituenten jeweils infrage kommen: Halogen, Cyano, Nitro, jeweils geradkettiges oder verzweigtes Alkyl, Alkoxy, Alkylthio, Halogenalkyl, Halogenalkoxy, Halogenalkylthio, Alkoxycarbonyl oder Alkoximinoalkyl mit jeweils 1 bis 4 Kohlenstoffatomen in den einzelnen Alkylteilen und gegebenenfalls 1 bis 9 gleichen oder verschiedenen Halogenatomen;
$X^2$ für eine elektronenanziehende Abgangsgruppe steht,
gegebenenfalls in Gegenwart eines Verdünnungsmittels und gegebenenfalls in Gegenwart eines Säurebindemittels umsetzt; oder
(c) die nach Verfahren (a) erhältlichen Aminomethyltetrahydrofurane der Formel (Ib)

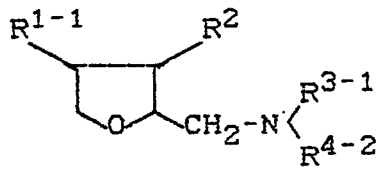

(Ib)

in welcher
$R^{1-1}$ für gegebenenfalls einfach bis mehrfach, gleich oder verschieden substituiertes Phenyl, wobei als Substituenten infrage kommen: Halogen, Cyano, Nitro, jeweils geradkettiges oder verzweigtes Alkyl, Alkoxy, Alkylthio, Halogenalkyl, Halogenalkoxy oder Halogenalkylthio mit jeweils 1 bis 6 Kohlenstoffatomen und gegebenenfalls 1 bis 9 gleichen oder verschiedenen Halogenatomen, Amino, jeweils geradkettiges oder verzweigtes Alkylamino, Dialkylamino oder Alkoximinoalkyl mit jeweils 1 bis 4 Kohlenstoffatomen in den einzelnen Alkylteilen;
ferner für jeweils gegebenenfalls einfach bis mehrfach, gleich oder verschieden substituiertes α-Naphthyl oder β-Naphthyl, wobei als Substituenten jeweils infrage kommen: Halogen, jeweils geradkettiges oder verzweigtes Alkyl oder Alkoxy mit jeweils 1 bis 4 Kohlenstoffatomen;
$R^2$ für Wasserstoff oder Methyl;
$R^{3-1}$ und $R^{4-2}$ unabhängig voneinander für Wasserstoff; für jeweils geradkettiges oder verzweigtes Alkyl mit 1 bis 12 Kohlenstoffatomen, Hydroxyalkyl mit 2 bis 6 Kohlenstoffatomen, Alkoxyalkyl oder Dialkoxyalkyl mit jeweils 1 bis 8 Kohlenstoffatomen oder Hydroxyalkoxyalkyl mit 2 bis 8 Kohlenstoffatomen in den einzelnen Alkylteilen; für jeweils geradkettiges oder verzweigtes Dioxolanylalkyl, Oxolanylalkyl oder Dioxanylalkyl mit jeweils 1 bis 4 Kohlenstoffatomen im Alkylteil oder für jeweils gegebenenfalls im Cycloalkylteil einfach oder mehrfach, gleich oder verschieden substituiertes Cycloalkyl oder Cycloalkylalkyl mit jeweils 3 bis 7 Kohlenstoffatomen im Cycloalkylteil und gegebenenfalls 1 bis 4 Kohlenstoffatomen im geradkettigen oder verzweigten Alkylteil stehen, wobei als Substituenten jeweils infrage kommen:
Halogen, jeweils geradkettiges oder verzweigtes Alkyl, Alkoxy, Halogenalkyl oder Halgoenalkoxy mit jeweils 1 bis 4 Kohlenstoffatomen und gegebenenfalls 1 bis 9 gleichen oder verschiedenen Halogenatomen; außerdem für jeweils gegebenenfalls einfach bis mehrfach, gleich oder verschieden im Arylteil substituiertes, Arylalkyl oder Aryl mit jeweils 6 bis 10 Kohlenstoffatomen im Arylteil und gegebenenfalls mit bis zu 6 Kohlenstoffatomen im geradkettigen oder verzweigten Alkylteil, wobei als Arylsubstituenten jeweils die oben genannten Cycloalkylsubstituenten infrage kommen; oder
$R^{3-1}$ und $R^{4-2}$ gemeinsam mit dem Stickstoffatom, an welches sie gebunden sind, für einen gegebenenfalls einfach bis mehrfach, gleich oder verschieden substituierten, gesättigten 5- bis 7-gliedrigen Heterocyclus, der gegebenenfalls ein weiteres Heteroatom, insbesondere Stickstoff, Sauerstoff oder Schwefel enthalten kann, wobei als Substituenten infrage kommen:
jeweils geradkettiges oder verzweigtes Alkyl oder Hydroxyalkyl mit jeweils 1 bis 4 Kohlenstoffatomen,

gegebenenfalls in Gegenwart eines Verdünnungsmittels, gegebenenfalls in Gegenwart eines Katalysators und gegebenenfalls unter erhöhtem Druck vorhandene Arylsubstituenten hydriert; und gegebenenfalls anschließend eine Säure addiert.

5. Schädlingsbekämpfungsmittel, gekennzeichnet durch einen Gehalt an mindestens einem 2-Aminomethyltetrahydrofuran der Formel (I) nach den Ansprüchen 1 und 4.

6. Verwendung von 2-Aminomethyltetrahydrofuran der Formel (I) nach den Ansprüchen 1 und 4 zur Bekämpfung von Schädlingen.

7. Verfahren zur Bekämpfung von Schädlingen, dadurch gekennzeichnet, daß man 2-Aminomethyltetrahydrofurane der Formel (I) nach den Ansprüchen 1 und 4 auf Schädlinge und/oder ihren Lebensraum einwirken läßt.

8. Verfahren zur Herstellung von Schädlingsbekämpfungsmitteln, dadurch gekennzeichnet, daß man 2-Aminomethyltetrahydrofurane der Formel (I) nach den Ansprüchen 1 und 4 mit Streckmitteln und/oder oberflächenaktiven Mitteln vermischt.

## Claims

1. 2-Aminomethyltetrahydrofurans of the general formula (I)

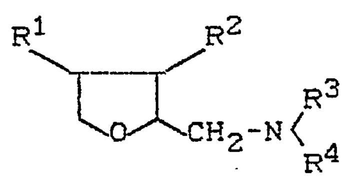

(I)

in which

$R^1$ represents phenyl which is optionally monosubstituted to polysubstituted by identical or different substituents, suitable substituents being: halogen, cyano, nitro, in each case straight-chain or branched alkyl, alkoxy, alkylthio, halogenoalkyl, halogenoalkoxy or halogenoalkylthio in each case having 1 to 6 carbon atoms and, if appropriate, 1 to 9 identical or different halogen atoms, amino, in each case straight-chain or branched alkylamino, dialkylamino or alkoximinoalkyl in each case having 1 to 4 carbon atoms in the individual alkyl parts; furthermore represents α-naphthyl or β-naphthyl which is in each case optionally monosubstituted to polysubstituted by identical or different substituents, suitable substituents in each case being: halogen, in each case straight-chain or branched alkyl or alkoxy in each case having 1 to 4 carbon atoms; furthermore represents cycloalkyl having 3 to 7 carbon atoms which is optionally monosubstituted to polysubstituted by identical or different substituents, suitable substituents being: in each case straight-chain or branched alkyl, alkoxy, halogenoalkyl or halogenoalkoxy in each case having 1 to 6 carbon atoms and, if appropriate, 1 to 9 identical or different halogen atoms; furthemore represents tetrahydronaphthyl or decahydronaphthyl which is in each case optionally monosubstituted to polysubstituted by identical or different substituents, suitable substituents for the hydrogenated rings being: straight-chain or branched alkyl and alkoxy in each case having 1 to 6 carbon atoms, and, in addition in the case of the aromatic rings, halogen; and furthermore represents a 5- or 6-membered heteroaryl radical having 1 or 2 heteroatoms, which is optionally monosubstituted to polysubstituted by identical or different alkyl substituents having 1 to 6 carbon atoms and/or halogen;

$R^2$ represents hydrogen or methyl, and

$R^3$ and $R^4$, independently of one another, in each case represent hydrogen; in each case straight-chain or branched alkyl having 1 to 12 carbon atoms, alkenyl having 3 to 8 carbon atoms, alkinyl having 3 to 8 carbon atoms, hydroxyalkyl having 2 to 6 carbon atoms, alkoxyalkyl or dialkoxyalkyl in each case having 1 to 8 carbon atoms, or hydroxyalkoxyalkyl having 2 to 8 carbon atoms in the individual alkyl parts; in each case straight-chain or branched dioxolanylalkyl, oxolanylalkyl or dioxanylalkyl in each case having 1 to 4 carbon atoms in the alkyl part, or cycloalkyl or cycloalkylalkyl which has in each case 3 to 7 carbon atoms in the cycloalkyl part and, if appropriate, 1 to 4 carbon atoms in the straight-chain or branched alkyl part and is in each case optionally monosubstituted to polysubstituted in the cycloalkyl part by identical or different substituents, suitable substituents in each case being: halogen, in each case straight-chain or branched alkyl, alkoxy, halogenoalkyl or halogenoalkoxy in each case having 1 to 4 carbon atoms and, if appropriate, 1 to 9 identical or different halogen atoms; in addition represent arylalkyl, arylalkenyl or aryl which has in each case 6 to 10 carbon atoms in the aryl part and, if appropriate, up to 6 carbon atoms in the straight-chain or branched alkyl or alkenyl part and which is in each case optionally monosubstituted to polysubstituted in the aryl part by identical or different substituents, suitable aryl substituents in each case being: halogen, cyano, nitro, in each case straight-chain or branched alkyl, alkoxy, alkylthio, halogenoalkyl, halogenoalkoxy, halogenoalkylthio or alkoximinoalkyl in each case having 1 to 4 carbon atoms in the individual alkyl parts and, if appropriate, 1 to 9 identical or different halogen atoms, or

$R^3$ and $R^4$, together with the nitrogen atom to which they are bound, represent a saturated heterocyclic ring which may optionally be substituted and which may optionally contain further heteroatoms, suitable

substituents being: in each case straight-chain or branched alkyl or hydroxyalkyl in each case having 1 to 4 carbon atoms, their acid addition salts, their geometrical and optical isomers, and their isomeric mixtures.

2. 2-Aminomethyltetrahydrofurans according to Claim 1, where, in the formula (I),
$R^1$ represents phenyl which is in each case optionally monosubstituted, disubstituted or trisubstituted by identical or different substituents, suitable substituents being: fluorine, chlorine, bromine, iodine, cyano, nitro, methyl, ethyl, n- or i-propyl, n-, i-, s- or t-butyl, n- or i-pentyl, n- or i-hexyl, methoxy, ethoxy, t-butoxy, trifluoromethyl, trifluoromethylthio, trifluoromethoxy, methoximinomethyl, dimethylamino and diethylamino; furthermore represents α-naphthyl or β-naphthyl which is in each case optionally monosubstituted, disubstituted or trisubstituted by identical or different substituents, suitable substituents in each case being: fluorine, chlorine, bromine, iodine, methyl, ethyl, n- or i-propyl, n-, i-, s- or t-butyl, methoxy and ethoxy;
furthermore represents cyclopentyl or cyclohexyl which is in each case optionally monosubstituted, disubstituted or trisubstituted by identical or different substituents, suitable substituents in each case being: methyl, ethyl, n- or i-propyl, n-, i-, s- or t-butyl, n- or i-pentyl, methoxy, ethoxy, t-butoxy, trifluoromethyl and difluoromethoxy;
furthermore represents tetrahydronaphthyl which is optionally monosubstituted, disubstituted or trisubstituted by identical or different substituents, suitable substituents being: fluorine, chlorine, bromine, methyl, ethyl, n- or i-propyl, n-, i-, s- or t-butyl, methoxy and ethoxy;
furthermore represents decahydronaphthyl which is optionally monosubstituted, disubstituted or trisubstituted by identical or different substituents, suitable substituents being: methyl, ethyl, n- or i-propyl, n-, i-, s- or t-butyl, methoxy and ethoxy;
or represents 2-, 3- or 4-pyridyl and 2- or 3-thienyl which is in each case optionally monosubstituted, disubstituted or trisubstituted by identical or different substituents, suitable substituents in each case being: fluorine, chlorine, bromine, methyl, ethyl, n-, or i-propyl, n-, i-, s- and t-butyl;
$R^2$ represents hydrogen or methyl, and
$R^3$ and $R^4$, independently of one another, in each case represent hydrogen, methyl, ethyl, n- or i-propyl, n- i-, s- or t-butyl, n- or i-pentyl, n- or i-hexyl, n- or i-heptyl, n- or i-octyl, allyl, n- or i-butenyl, n- or i-pentenyl, propargyl, n- or i-butinyl, hydroxyethyl, hydroxyropyl, methoxyethyl, ethoxyethyl, propoxyethyl, butoxyethyl, methoxypropyl, ethoxypropyl, propoxypropyl, butoxypropyl, hydroxyethoxyethyl, dimethoxyethyl, dimethoxypropyl, diethoxyethyl, dioxolanylmethyl, dioxolanylethyl, oxolanylmethyl, oxolanylethyl, dioxanylmethyl or dioxanylethyl or represents cyclopropyl, cyclopropylmethyl, cyclopropylethyl, cyclopropylpropyl, cyclopentyl, cyclopentylmethyl, cyclohexyl or cyclohexylmethyl, which is in each case optionally monosubstituted to pentasubstituted by identical or different substituents from amongst fluorine, chlorine, bromine, methyl, ethyl, n- or i-propyl, n-, i-, s- or t-butyl, or represents phenyl, benzyl or phenylethyl which is in each case optionally monosubstituted, disubstituted or trisubstituted by identical or different substituents, suitable substituents in each case being: fluorine, chlorine, bromine, cyano, nitro, methyl, ethyl, n- or i-propyl, n-, i-, s- or t- butyl, methoxy, ethoxy, methylthio, trifluoromethoxy, trifluoromethylthio, methoxycarbonyl or methoximinomethyl, their acid-addition salts, their geometrical and optical isomers, and their isomeric mixtures.

3. 2-Aminomethyltetrahydrofurans according to Claim 1, where, in the formula (I),
$R^1$ represents phenyl which is in each case optionally monosubstituted, disubstituted or trisubstituted by identical or different substitutents, suitable substituents being: fluorine, chlorine, bromine, iodine, cyano, nitro, methyl, ethyl, ni-propyl, n-, i-, s- or t-butyl, n- or i-pentyl, n- or i-hexyl, methoxy, ethoxy, t-butoxy, trifluoromethyl, trifluoromethylthio, trifluoromethoxy, methoximinomethyl, dimethylamino and diethylamino; furthermore represents α-naphthyl or β-naphthyl which is in each case optionally monosubstituted, disubstituted or trisubstituted by identical or different substituents, suitable substituents in each case being: fluorine, chlorine, bromine, iodine, methyl, ethyl, n- or i-propyl, n-, i-, s- or t-butyl, methoxy and ethoxy;
furthermore represents cyclopentyl or cyclohexyl which is in each case optionally monosubstituted, disubstituted or trisubstituted by identical or different substituents, suitable substituents in each case being; methyl, ethyl, n- or i-propyl, n-, i-, s- or t-butyl, n- or i-pentyl, methoxy, ethoxy, t-butoxy, trifluoromethyl and difluoromethoxy;
furthermore represents tetrahydronaphthyl which is optionally monosubstituted, disubstituted or trisubstituted by identical or different substituents, suitable substituents being: fluorine, chlorine, bromine, methyl, ethyl, n- or i-propyl, n-, i-, s- or t-butyl, methoxy and ethoxy;
furthermore represents decahydronaphthyl which is optionally monosubstituted, disubstituted or trisubstituted by identical or different substituents, suitable substituents being: methyl, ethyl, n- or i-propyl, n-, i-, s- or t-butyl, methoxy and ethoxy;
or represents 2-, 3- or 4-pyridyl and 2- or 3-thienyl which is in each case optionally monosubstituted, disubstituted or trisubstituted by identical or different substituents, suitable substituents in each case being: fluorine, chlorine, bromine, methyl, ethyl, n- or i-propyl, n-, i-, s- and t-butyl;
$R^2$ represents hydrogen or methyl, and
$R^3$ and $R^4$, together with the nitrogen atom to which they are bound, represent a heterocyclic ring of the formula

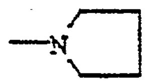 ; 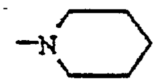 ; 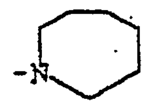 ; 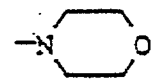

or 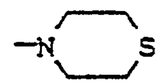

which is optionally monosubstituted, disubstituted or trisubstituted by identical or different substituents, suitable substituents being: methyl, ethyl or hydroxymethyl, their acid-addition salts, their geometrical and optical isomers, and their isomeric mixtures.

4. Process for the preparation of 2-aminomethyltetrahydrofurans of the general formula (I)

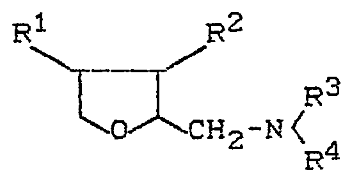 (I)

in which

$R^1$ represents phenyl which is optionally monosubstituted to polysubstituted by identical or different substituents, suitable substituents being: halogen, cyano, nitro, in each case straight-chain or branched alkyl, alkoxy, alkylthio, halogenalkyl, halogenoalkoxy or halogenoalkylthio in each case having 1 to 6 carbon atoms and, if appropriate, 1 to 9 identical or different halogen atoms, amino, in each case straight-chain or branched alkylamino, dialkylamino or alkoximinoalkyl in each case having 1 to 4 carbon atoms in the individual alkyl parts; furthermore represents α-naphthyl or β-naphthyl which is in each case optionally monosubstituted to polysubstituted by identical or different substituents, suitable substituents in each case being: halogen, in each case straight-chain or branched alkyl or alkoxy in each case having 1 to 4 carbon atoms; furthemore represents cycloalkyl having 3 to 7 carbon atoms which is optionally monosubstituted to polysubstituted by identical or different substituents, suitable substituents being: in each case straight-chain or branched alkyl, alkoxy, halogenoalkyl or halogenoalkoxy in each case having 1 to 6 carbon atoms and, if appropriate, 1 to 9 identical or different halogen atoms; furthermore represents tetrahydronaphthyl or decahydronaphthyl which is in each case optionally monosubstituted to polysubstituted by identical or different substituents, suitable substituents for the hydrogenated rings being: straight-chain or branched alkyl and alkoxy in each case having 1 to 6 carbon atoms, and, in addition in the case of the aromatic rings, halogen; and furthermore represents a 5- or 6-membered heteroaryl radical having 1 or 2 heteroatoms, which is optionally monosubstituted to polysubstituted by identical or different alkyl substituents having 1 to 6 carbon atoms and/or halogen;

$R^2$ represents hydrogen or methyl, and

$R^3$ and $R^4$, independently of one another, in each case represent hydrogen; in each case straightchain or branched alkyl having 1 to 12 carbon atoms, alkenyl having 3 to 8 carbon atoms, alkinyl having 3 to 8 carbon atoms, hydroxyalkyl having 2 to 6 carbon atoms, alkoxyalkyl or dialkoxyalkyl in each case having 1 to 8 carbon atoms, or hydroxyalkoxyalkyl having 2 to 8 carbon atoms in the individual alkyl parts; in each case straight-chain or branched dioxolanylalkyl, oxolanylalkyl or dioxanylalkyl in each case having 1 to 4 carbon atoms in the alkyl part, or cycloalkyl or cycloalkylalkyl which has in each case 3 to 7 carbon atoms in the cycloalkyl part and, if appropriate, 1 to 4 carbon atoms in the straight-chain or branched alkyl part and is in each case optionally monosubstituted to polysubstituted in the cycloalkyl part by identical or different substituents, suitable substituents in each case being: halogen, in each case straight-chain or branched alkyl, alkoxy, halogenoalkyl or halogenoalkoxy in each case having 1 to 4 carbon atoms and, if appropriate, 1 to 9 identical or different halogen atoms; in addition represent arylalkyl, arylalkenyl or aryl which has in each case 6 to 10 carbon atoms in the aryl part and, if appropriate, up to 6 carbon atoms in the straight-chain or branched alkyl or alkenyl part and which is in each case optionally monosubstituted to polysubstituted in the aryl part by identical or different substituents, suitable aryl substituents in each case being: halogen, cyano, nitro, in each case straight-chain or branched alkyl, alkoxy, alkylthio, halogenoalkyl, halogenoalkoxy, halogenoalkylthio or alkoximinoalkyl in each case having 1 to 4 carbon atoms in the individual alkyl parts and, if appropriate, 1 to 9 identical or different halogen atoms, or

$R^3$ and $R^4$, together with the nitrogen atom to which they are bound, represent a saturated heterocyclic ring which may optionally be substituted and which may optionally contain further heteroatoms, suitable substituents being: in each case straight-chain or branched alkyl or hydroxyalkyl in each case having 1 to 4 carbon atoms, their acid addition salts, their geometrical and optical isomers, and their isomeric mixtures, characterized in that

(a) substituted tetrahydrofurans of the formula (II)

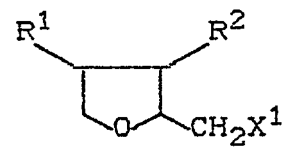

(II)

in which

$R^1$ and $R^2$ have the abovementioned meaning, and

$X^1$ represents an electron-withdrawing leaving group, are reacted with amines of the formula (III)

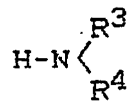

(III)

in which

$R^3$ and $R^4$ have the abovementioned meaning, if appropriate in the presence of a diluent and if appropriate in the presence of an acid-binding agent; or in that

(b) the aminomethyltetrahydrofurans of the formula (Ia)

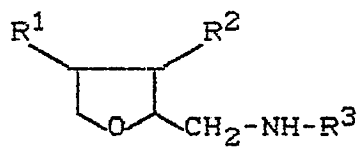

(Ia)

in which

$R^1$, $R^2$ and $R^3$ have the abovementioned meaning, obtainable by process (a) and/or (c) are reacted with alkylating agents of the formula (IV)

$$R^{4-1}-X^2 \qquad (IV)$$

in which

$R^{4-1}$ represents in each case straight-chain or branched alkyl having 1 to 12 carbon atoms, alkenyl having 3 to 8 carbon atoms, alkinyl having 3 to 8 carbon atoms, hydroxyalkyl having 2 to 6 carbon atoms, alkoxyalkyl, dialkoxyalkyl or hydroxyalkoxyalkyl in each case having 1 to 6 carbon atoms in the individual alkyl parts, dioxolanylalkyl, oxolanylalkyl or dioxanylalkyl in each case having 1 to 4 carbon atoms in the straight-chain or branched alkyl part, or cycloalkyl or cycloalkylalkyl which has in each case 3 to 7 carbon atoms in the cycloalkyl part and, if appropriate, 1 to 4 carbon atoms in the straight-chain or branched alkyl part and is in each case optionally monosubstituted to polysubstituted in the cycloalkyl part by identical or different substituents, suitable substituents in each case being: halogen, in each case straight-chain or branched alkyl, alkoxy, halogenoalkyl or halogenoalkoxy in each case having 1 to 4 carbon atoms and, if appropriate, 1 to 9 identical or different halogen atoms; in addition represents arylalkyl or arylalkenyl which has in each case 6 to 10 carbon atoms in the aryl part and up to 6 carbon atoms in the straight-chain or branched alkyl or alkenyl part and which is in each case optionally monosubstituted to polysubstituted in the aryl part by identical or different substituents, suitable aryl substituents in each case being: halogen, cyano, nitro, in each case straight-chain or branched alkyl, alkoxy, alkylthio, halogenoalkyl, halogenoalkoxy, halogenoalkylthio, alkoxycarbonyl or alkoximinoalkyl in each case having 1 to 4 carbon atoms in the individual alkyl parts and, if appropriate, 1 to 9 identical or different halogen atoms; and

$X^2$ represents an electron-withdrawing leaving group, if appropriate in the presence of a diluent and if appropriate in the presence of an acid-binding agent; or in that

(c) the aminomethyltetrahydrofurans of the formula (Ib)

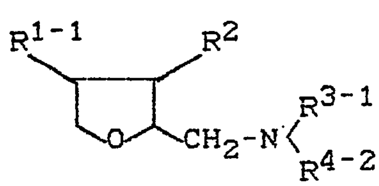

(Ib)

in which

$R^{1-1}$ represents phenyl which is optionally monosubstituted to polysubstituted by identical or different substituents, suitable substituents being: halogen, cyano, nitro, in each case straight-chain or branched alkyl, alkoxy, alkylthio, halogenoalkyl, halogenoalkoxy or halogenoalkylthio in each case having 1 to 6 carbon atoms and, if appropriate, 1 to 9 identical or different halogen atoms, amino, in each case straight-chain or branched alkylamino, dialkylamino or alkoximinoalkyl in each case having 1 to 4 carbon atoms in the individual alkyl parts; furthermore represents α-naphthyl or β-naphthyl which is in each case optionally monosubstituted to polysubstituted by identical or different substituents, suitable substituents in each case being: halogen, in each case straight-chain or branched alkyl or alkoxy in each case having 1 to 4 carbon atoms;
$R^2$ represents hydrogen or methyl; and
$R^{3-1}$ and $R^{4-2}$, independently of one another, in each case represent hydrogen; in each case straight-chain or branched alkyl having 1 to 12 carbon atoms, hydroxyalkyl having 2 to 6 carbon atoms, alkoxyalkyl or dialkoxyalkyl in each case having 1 to 8 carbon atoms, or hydroxyalkoxyalkyl having 2 to 8 carbon atoms in the individual alkyl parts; in each case straight-chain or branched dioxolanylalkyl, oxolanylalkyl or dioxanylalkyl in each case having 1 to 4 carbon atoms in the alkyl part, or cycloalkyl or cycloalkylalkyl which has in each case 3 to 7 carbon atoms in the cycloalkyl part and, if appropriate, 1 to 4 carbon atoms in the straight-chain or branched alkyl part and is in each case optionally monosubstituted to polysubstituted in the cycloalkyl part by identical or different substituents, suitable substituents in each case being: halogen, in each case straight-chain or branched alkyl, alkoxy, halogenoalkyl or halogenoalkoxy in each case having 1 to 4 carbon atoms and, if appropriate, 1 to 9 identical or different halogen atoms; in addition represent arylalkyl, or aryl which has in each case 6 to 10 carbon atoms in the aryl part and, if appropriate, up to 6 carbon atoms in the straight-chain or branched alkyl part and which is in each case optionally monosubstituted to polysubstituted in the aryl part by identical or different substituents, suitable aryl substituents in each case being the cycloalkyl substituents mentioned above; or
$R^{3-1}$ and $R^{4-2}$, together with the nitrogen atom to which they are bound, represent a saturated 5- to 7-membered heterocyclic ring which may optionally be monosubstituted to polysubstituted by identical or different substituents, suitable substituents being: in each case straight-chain or branched alkyl or hydroxyalkyl in each case having 1 to 4 carbon atoms, and which heterocyclic ring may optionally contain a further heteroatom, in particular nitrogen, oxygen or sulphur, obtainable by process (a), are hydrogenated on the aryl substituents, if appropriate in the presence of a diluent and if appropriate in the presence of a catalyst and if appropriate under increased pressure; and, if appropriate, acid is subsequently added.

5. Pesticides, characterized in that they contain at least one 2-aminomethyltetrahydrofuran of the formula (I) according to Claims 1 and 4.

6. Use of 2-aminomethyltetrahydrofuran of the formula (I) according to Claims 1 and 4 for combating pests.

7. Process for combating pests, characterized in that 2-aminomethyltetrahydrofurans of the formula (I) according to Claims 1 and 4 are allowed to act on the pests and/or on their habitat.

8. Process for the preparation of pesticides, characterized in that 2-aminomethyltetrahydrofurans of the formula (I) according to Claims 1 and 4 are mixed with extenders and/or surface-active agents.

**Revendications**

1. 2-aminométhyltétrahydrofurannes de formule générale I

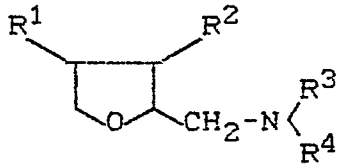

(I)

dans laquelle
$R^1$ représente un groupe phényle portant, le cas échéant, un ou plusieurs substituants identiques ou différents choisis parmi les halogènes, les groupes cyano, nitro, les groupes alkyle, alcoxy, alkylthio, halogénoalkyle, halogénoalcoxy ou halogénoalkylthio à chaîne droite ou ramifiée contenant chacun 1 à 6 atomes de carbone et, le cas échéant, 1 à 9 atomes d'halogènes identiques ou différents, les groupes amino, les groupes alkylamino, dialkylamino, ou alcoximinoalkyle à chaîne droite ou ramifiée contenant chacun 1 à 4 atomes de carbone dans les diverses parties alkyle;
ou encore un groupe α-naphtyle ou β-naphtyle portant éventuellement un à plusieurs substituants identiques ou différents choisis parmi les haogènes, les groupes alkyle ou alcoxy à chaîne droite ou ramifiée contenant 1 à 4 atomes de carbone;
ou encore un groupe cycloalkyle en $C_3$–$C_7$ portant éventuellement un ou plusieurs substituants identiques ou différents choisis parmi les groupes alkyle, alcoxy, halogénoalkyle ou halogénoalcoxy à chaîne

droite ou ramifiée contenant chacun 1 à 6 atomes de carbone et, le cas échéant, 1 à 9 atomes d'halogènes identiques ou différents;
ou encore un groupe tétrahydronaphtyle ou décahydronaphtyle portant éventuellement un ou plusieurs substituants identiques ou différents choisis parmi les groupes alkyle et alcoxy contenant chacun 1 à 6 atomes de carbone pour les noyaux hydrogénés, et en outre parmi les halogènes pour les noyaux aromatiques; ou encore un groupe hétéroaryle à 5 ou 6 chaînons et un ou deux hétéroatomes portant éventuellement un ou plusieurs substituants identiques ou différents choisis parmi les groupes alkyle en $C_1$–$C_6$ et/ou les halogènes,
$R^2$ représente l'hydrogène ou un groupe méthyle, et
$R^3$ et $R^4$ représentent chacun, indépendamment l'un de l'autre, l'hydrogène; un groupe à chaîne droite ou ramifiée alkyle en $C_1$–$C_{12}$, alcényle en $C_3$–$C_8$, alcynyle en $C_3$–$C_8$, hydroxyalkyle en $C_2$–$C_6$, alcoxyalkyle ou dialcoxyalkyle contenant chacun 1 à 8 atomes de carbone ou hydroxyalcoxyalkyle contenant 2 à 8 atomes de carbone dans les diverses parties alkyle; un groupe à chaîne droite ou ramifiée dioxolannylalkyle, oxolannylalkyle ou dioxannylalkyle contenant chacun 1 à 4 atomes de carbone dans la partie alkyle, ou un groupe cycloalkyle ou cycloalkylalkyle contenant chacun 3 à 7 atomes de carbone dans la partie cycloalkyle et, le cas échéant, 1 à 4 atomes de carbone dans la partie alkyle à chaîne droite ou ramifiée, et portant, le cas échéant, dans la partie cycloalkyle un ou plusieurs substituants identiques ou différents choisis parmi:
les halogènes, les groupes à chaîne droite ou ramifiée alkyle, alcoxy, halogénoalkyle ou halogénoalcoxy contenant chacun 1 à 4 atomes de carbone et, le cas échéant, 1 à 9 atomes d'halogènes identiques ou différents; ou bien un groupe arylalkyle, arylalcényle ou aryle contenant chacun 6 à 10 atomes de carbone dans la partie aryle et, le cas échéant, jusqu'à 6 atomes de carbone dans la partie alkyle ou alcényle à chaîne droite ou ramifiée, et portant, le cas échéant, dans la partie aryle un ou plusieurs substituants identiques ou différents choisis parmi:
les halogènes, les groupes cyano, nitro, les groupes alkyle, alcoxy, alkylthio, halogénoalkyle, halogénoalcoxy, halogénoalkylthio ou alcoximinoalkyle à chaîne droite ou ramifiée contenant 1 à 4 atomes de carbone dans les diverses parties alkyle et, le cas échéant, 1 à 9 atomes d'halogènes identiques ou différents, ou bien
$R^3$ et $R^4$ forment ensemble et avec l'atome d'azote auquel ils sont reliés un hétérocycle saturé éventuellement substitué et qui peut contenir, le cas échéant, d'autres hétéroatomes, les substituants en question étant: des groupes alkyle ou hydroxyalkyle à chaîne droite ou ramifiée en $C_1$–$C_4$,
leurs sels formés par addition avec des acides, leurs isomères géométriques et optiques et leurs mélanges d'isomères.

2. 2-aminométhyltétrahydrofurannes selon la revendication 1, répondant à la formule I, dans laquelle:
$R^1$ représente un groupe phényle portant éventuellement un à trois substituants identiques ou différents choisis parmi le fluor, le chlore, le brome, l'iode, les groupes cyano, nitro, méthyle, éthyle, n-, i-propyle, n-, i-, s-, t-butyle, n-, i-pentyle, n-, i-hexyle, méthoxy, éthoxy, tert-butoxy, trifluorométhyle, trifluorométhylthio, trifluorométhoxy, méthoximinométhyle, diméthylamino et diéthylamino;
ou encore un groupe $\alpha$-naphtyle ou $\beta$-naphtyle portant, le cas échéant, un à trois substituants identiques ou différents choisis parmi le fluor, le chlore, le brome, l'iode, les groupes méthyle, éthyle, n-, i-propyle, n-, i-, s-, t-butyle, méthoxy et éthoxy;
ou encore un groupe cyclopentyle ou cyclohexyle portant, le cas échéant, un à trois substituants identiques ou différents choisis parmi les groupes méthyle, éthyle, n-, i-propyle-, n-, i-, s-, t-butyle, n-, i-pentyle, méthoxy, éthoxy, t-butoxy, trifluorométhyle et difluorométhoxy;
ou encore un groupe tétrahydronaphtyle portant, le cas échéant, un à trois substituants identiques ou différents choisis parmi le fluor, le chlore, le brome, les groupes méthyle, éthyle, n-, i-propyle, n-, i-, s-, t-butyle, méthoxy et éthoxy;
ou encore un groupe décahydronapthyle portant éventuellement un à trois substituants identiques ou différents choisis parmi les groupes méthyle, éthyle, n-, i-propyle, n-, i-, s-, t-butyle, méthoxy et éthoxy;
ou encore un groupe 2-, 3- ou 4-pyridyle ou 2- ou 3-thiényle portant, le cas échéant, un à trois substituants identiques ou différents choisis parmi le fluor, le chlore, le brome, les groupes méthyle, éthyle, n-, i-propyle, n-, i-, s- et t-butyle;
$R^2$ représente l'hydrogène ou un groupe méthyle; et
$R^3$ et $R^4$ représentent chacun, indépendamment l'un de l'autre, l'hydrogène, un groupe méthyle, éthyle, n- ou i-propyle, n-, i-, s- ou t-butyle, n- ou i-pentyle, n- ou i-hexyle, n- ou i-heptyle, n- ou i-octyle, allyle, n- ou i-butényle, n- ou i-pentényle, propargyle, n- ou i-butynyle, hydroxyéthyle, hydroxypropyle, méthoxyéthyle, éthoxyéthyle, propoxyéthyle, butoxyéthyle, méthoxypropyle, éthoxypropyle, propoxypropyle, butoxypropyle, hydroxyéthoxyéthyle, diméthoxyéthyle, diméthoxypropyle, diéthoxyéthyle dioxolannylméthyle, dioxolannyléthyle, oxolannylméthyle, oxolannyléthyle dioxannylméthyle, dioxannyléthyle, un groupe cyclopropyle, cyclopropylméthyle, cyclopropyléthyle, cyclopropylpropyle, cyclopentyle, cyclopentylméthyle, cyclohexyle ou cyclohexylméthyle portant éventuellement un à cinq substituants identiques ou différents choisis parmi le fluor, le chlore, le brome, les groupes méthyle, éthyle, n- ou i-propyle, n-, i-, s- ou t-butyle, ou encore un groupe phényl, benzyle ou phényléthyle portant, le cas échéant, un à trois substituants identiques ou différents choisis parmi le fluor, le chlore, le brome, les groupes cyano, nitro, méthyle, éthyle, n- ou i-propyle, n-, i-, s- ou t-butyle, méthoxy, éthoxy, méthylthio,

trifluorométhoxy, trifluorométhylthio, méthoxycarbonyle ou méthoximinométhyle,
leurs sels formés par addition avec des acides, leurs isomères géométriques et optiques et leurs mélanges d'isomères.

3. 2-aminométhyltétrahydrofurannes selon la revendication 1, répondant à la formule I, dans laquelle:
$R^1$ représente un groupe phényle portant éventuellement un à trois substituants identiques ou différents choisis parmi le fluor, le chlore, le brome, l'iode, les groupes cyano, nitro, méthyle, éthyle, n-, i-propyle, n-, i-, s-, t-butyle, n-, i-pentyle, n-, i-hexyle, méthoxy, éthoxy, t-butoxy, trifluorométhyle, trifluorométhylthio, trifluorométhoxy, méthoximinométhyle, diméthylamino et diéthylamino;
ou encore un groupe α-naphtyle ou β-naphtyle portant éventuellement un à trois substituants identiques ou différents choisis parmi le fluor, le chlore, le brome, l'iode, les groupes méthyle, éthyle, n- et i-propyle, n-, i-, s-, t-butyle, méthoxy et éthoxy;
ou encore un groupe cyclopentyle ou cyclohexyle portant éventuellement un à trois substituants identiques ou différents choisis parmi les groupes méthyle, éthyle, n- et i-propyle, n-, i-, s-, t-butyle, n-, i-pentyle, méthoxy, éthoxy, t-butoxy, trifluorométhyle et difluorométhoxy;
ou encore un groupe tétrahydronaphtyle portant éventuellement un à trois substituants identiques ou différents choisis parmi le fluor, le chlore, le brome, les groupes méthyle, éthyle, n- et i-propyle, n-, i-, s-, t-butyle, méthoxy et éthoxy;
ou encore un groupe décahydronaphtyle portant éventuellement un à trois substituants identiques ou différents choisis parmi les groupes méthyle, éthyle, n- et i-propyle, n-, i-, s-, t-butyle, méthoxy et éthoxy;
ou encore un groupe 2-, 3- ou 4-pyridyle ou 2- ou 3-thiényle portant éventuellement un à trois substituants identiques ou différents choisis parmi le fluor, le chlore, le brome, les groupes méthyle, éthyle, n- et i-propyle, n-, i-, s- et t-butyle;
$R^2$ représente l'hydrogène ou un groupe méthyle, et
$R^3$ et $R^4$ forment ensemble et avec l'atome d'azote auquel ils sont reliés un hétérocycle de formule:

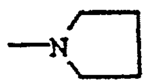 ; 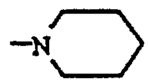 ; 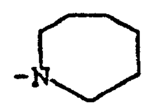 ; 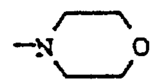

ou 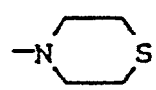

portant éventuellement un à trois substituants identiques ou différents choisis parmi les groupes méthyle, éthyle ou hydroxyméthyle, leurs sels formés par addition avec des acides, leurs isomères géométriques et optiques et leurs mélanges d'isomères.

4. Procédé de préparation des 2-aminométhyltétrahydrofurannes de formule générale I:

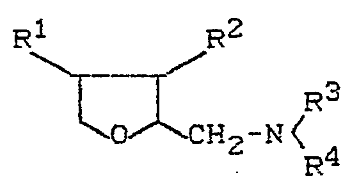 (I)

dans laquelle
$R^1$ représente un groupe phényle portant éventuellement un à trois substituants identiques ou différents choisis parmi les halogènes, les groupes cyano, nitro, les groupes alkyle, alcoxy, alkylthio, halogénoalkyle, halogénoalcoxy ou halogénoalkylthio à chaîne droite ou ramifiée contenant chacun 1 à 6 atomes de carbone et, le cas échéant, 1 à 9 atomes d'halogènes identiques ou différents, les groupes amino, les groupes alkylamino, dialkylamino ou alcoximinoalkyle à chaîne droite ou ramifiée contenant chacun 1 à 4 atomes de carbone dans les diverses parties alkyle;
ou encore un groupe α-naphtyle ou β-naphtyle portant, le cas échéant, un ou plusieurs substituants choisis parmi les halogènes, les groupes alkyle ou alcoxy à chaîne droite ou ramifiée contenant chacun 1 à 4 atomes de carbone;
ou encore un groupe cycloalkyle en $C_3$–$C_7$ portant, le cas échéant, un ou plusieurs substituants identiques ou différents choisis parmi les groupes alkyle, alcoxy, halogénoalkyle ou halogénoalcoxy à chaîne droite ou ramifiée contenant chacun 1 à 6 atomes de carbone et, le cas échéant, 1 à 9 atomes d'halogènes identiques ou différents;
ou encore un groupe tétrahydronaphtyle ou décahydronaphtyle portant, le cas échéant, un ou plusieurs substituants identiques ou différents choisis parmi:
les groupes alkyle et alcoxy à chaîne droite ou ramifiée en $C_1$–$C_6$ pour les noyaux hydrogénés et, en

outre, les halogènes pour les noyaux aromatiques;
ou encore un groupe hétéroaryle à 5 ou 6 chaînons et un ou deux hétéroatomes portant éventuellement un ou plusieurs substituants choisis parmi les groupes alkyle en $C_1$–$C_6$ et/ou les halogènes,
$R^2$ représente l'hydrogène ou un groupe méthyle, et de leurs sels formés par addition avec des acides, de leurs isomères géométriques et optiques et de leurs mélanges d'isomères, caractérisé en ce que:
(a) on fait réagir des tétrahydrofurannes substitués de formule II:

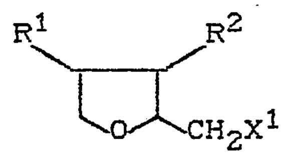
(II)

dans laquelle $R^1$ et $R^2$ ont les significations indiquées ci-dessus et,
$X^1$ représente un groupe éliminable électrophile, avec des amines de formule III

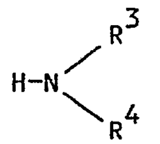
(III)

dans laquelle $R^3$ et $R^4$ ont les significations indiquées ci-dessus, éventuellement en présence d'un diluant et éventuellement en présence d'un accepteur d'acide; ou bien
(b) on fait réagir les aminométhyltétrahydrofurannes de formule Ia obtenue par les procédés (a) et/ou (c)

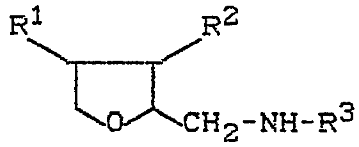
(Ia)

dans laquelle $R^1$, $R^2$ et $R^3$ ont les significations indiquées ci-dessus,
avec des agents alkylants de formule IV
$R^{4-1}$–$X^2$ (IV)
dans laquelle
$R^{4-1}$ représente un groupe, à chaîne droite ou ramifiée, alkyle en $C_1$–$C_{12}$, alcényle en $C_3$–$C_8$, alcynyle en $C_3$–$C_8$, hydroxyalkyle en $C_2$–$C_6$, alcoxyalkyle, dialcoxyalkyle ou hydroxyalcoxyalkyle contenant chacun 1 à 6 atomes de carbone dans les diverses parties alkyle, un groupe dioxolannylalkyle, oxolannylalkyle ou dioxannylalkyle contenant chacun 1 à 4 atomes de carbone dans la partie alkyle à chaîne droite ou ramifiée, un groupe cycloalkyle ou cycloalkylalkyle contenant chacun 3 à 7 atomes de carbone dans la partie cycloalkyle et, le cas échéant, 1 à 4 atomes de carbone dans la partie alkyle à chaîne droite ou ramifiée, et portant éventuellement dans la partie cycloalkyle un ou plusieurs substituants identiques ou différents choisis parmi: les halogènes, les groupes alkyle, alcoxy, halogénoalkyle ou halogénoalxoxy à chaîne droite ou ramifiée contenant 1 à 4 atomes de carbone et, les échéant, 1 à 9 atomes d'halogènes identiques ou différents; ou encore un groupe arylalkyle ou arylalcényle contenant chacun 6 à 10 atomes de carbone dans la partie aryle et jusqu'à 6 atomes de carbone dans la partie alkyle ou alcényle à chaîne droit ou ramifiée et portant éventuellement dans la partie aryle un ou plusieurs substituants identiques ou différents choisis parmi les halogènes, les groupes cyano, nitro, les groupes alkyle, alcoxy, alkylthio, halogénoalkyle, halogénoalcoxy, halogénoalkylthio, alcoxycarbonyle ou alcoximinoalkyle à chaîne droite ou ramifiée contenant chacun 1 à 4 atomes de carbone dans les diverses parties alkyle et, le cas échéant, 1 à 9 atomes d'halogènes identiques ou différents;
$X^2$ représente un groupe éliminable électrophile,
éventuellement en présence d'un diluant et éventuellement en présence d'un accepteur d'acide;
ou bien
(c) on hydrogène les substituants aryles présents dans les aminométhyltétrahydrofurannes de formule Ib obtenus par le procédé (a)

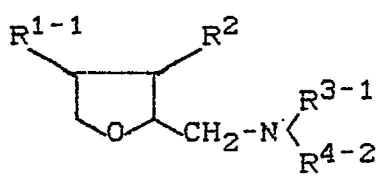
(Ib)

dans laquelle
$R^{1-1}$ représente un groupe phényle portant éventuellement un ou plusieurs substituants identiques ou différents choisis parmi les halogènes, les groupes cyano, nitro, les groupes alkyle, alcoxy, alkylthio, halogénoalkyle, halogénoalcoxy ou halogénoalkylthio à chaîne droite ou ramifiée contenant chacun 1 à 6 atomes de carbone et, le cas échéant, 1 à 9 atomes d'halogènes identiques ou différents, les groupes amino, les groupes alkylamino, dialkylamino ou alcoximinoalkyle à chaîne droite ou ramifiée contenant chacun 1 à 4 atomes de carbone dans les diverses parties alkyle; ou encore un groupe α-napthyle ou β-naphtyle portant éventuellement un ou plusieurs substituants identiques ou différents choisis parmi les halogènes, les groupes alkyle ou alcoxy à chaîne droite ou ramifiée en $C_1$–$C_4$;
$R^2$ représente l'hydrogène ou un grope méthyle;
$R^{3-1}$ et $R^{4-2}$ représentent chacun, indépendamment l'un de l'autre, l'hydrogène; un groupe à chaîne droite ou ramifiée alkyle en $C_1$–$C_{12}$, hydroxyalkyle en $C_2$–$C_6$, alcoxyalkyle ou dialcoxyalkyle contenant chacun 1 à 8 atomes de carbone ou hydroxyalcoxyalkyle en $C_2$–$C_8$ dans les diverses parties alkyle; un groupe dioxolannylalkyle, oxolannylalkyle ou dioxannylalkyle à chaîne droite ou ramifiée contenant chacun 1 à 4 atomes de carbone dans la partie alkyle, ou un groupe cycloalkyle ou cycloalkylalkyle contenant chacun 3 à 7 atomes de carbone dans la partie cycloalkyle et éventuellement 1 à 4 atomes de carbone dans la partie alkyle à chaîne droite ou ramifiée, et portant éventuellement dans la partie cycloalkyle un ou plusieurs substituants identiques ou différents choisis parmi les halogènes, les groupes alkyle, alcoxy, halogénoalkyle ou halogénoalcoxy à chaîne droite ou ramifiée contenant chacun 1 à 4 atomes de carbone et, le cas échéant, 1 à 9 atomes d'halogènes identiques ou différents; ou encore un groupe arylalkyle ou aryle contenant chacun 6 à 10 atomes de carbone dans la partie aryle et, le cas échéant, jusqu'à 6 atomes de carbone dans la partie alkyle à chaîne droite ou ramifiée et portant éventuellement dans la partie aryle un ou plusieurs substituants choisis parmi ceux mentionnés ci-dessus pour les groupes cycloalkyle; ou bien
$R^{3-1}$ et $R^{4-2}$ forment ensemble et avec l'atome d'azote auquel ils sont reliés un hétérocycle saturé de 5 à 7 chaînons portant éventuellement un autre hétéroatome, en particulier d'azote, d'oxygène ou de soufre, et portant éventuellement un ou plusieurs substituants identiques ou différents choisis parmi les groupes alkyle ou hydroxyalkyle à chaîne droite ou ramifiée en $C_1$–$C_4$,
éventuellement en présence d'un diluant, éventuellement en présence d'un catalyseur et éventuellement sous pression; et, le cas échéant, on fixe ensuite un acide par addition.

5. Produits pesticides, caractérisés en ce qu'ils contiennent au moins un 2-aminométhyltétrahydrofuranne de formule I selon les revendications 1 et 4.

6. Utilisation des 2-aminométhyltétrahydrofurannes de formule I selon les revendications 1 à 4 dans la lutte contre les parasites.

7. Procédé pour combattre les parasites, caractérisé en ce que l'on fait agir des 2-aminométhyltétrahydrofurannes de formule I selon les revendications 1 et 4 sur les parasites et/ou leur habitat.

8. Procédé de préparation de produits pesticides, caractérisé en ce que l'on mélange des 2-aminométhyltétrahydrofurannes de formule I selon les revendications 1 et 4 avec des diluants et/ou des agents tensioactifs.